# EUROPEAN PATENT SPECIFICATION

(11) **EP 0 413 329 B1**
(45) Date of publication and mention of the grant of the patent: **05.02.1997**
(21) Application number: 90115691.9
(22) Date of filing: 16.08.1990
(51) Int. Cl.: A61K 31/42, A61K 31/275

(54) **5-Methyl-isoxazole-4-carboxylic acid anilides and 2-hydroxyethylidene-cyano acetic acid anilides for the treatment of ocular diseases**
5-Methylisoxazol-4-carbonsäure-anilide und 2-Hydroxyethyliden-cyanoessigsäure-anilide zur Behandlung von Augenkrankheiten
Anilides de l'acide 5-méthyl-isoxazole-4-carboxylique et de l'acide 2-hydroxyéthylidène-cyanoacétique pour le traitement des maladies oculaires

(30) Priority: 18.08.1989 US 395860
(43) Date of publication of application: 20.02.1991
(73) Proprietor: ALCON LABORATORIES, INC., Fort Worth Texas 76134-2099 (US)
(72) Inventor: Robertson, Stella M., Arlington, Texas 76016 (US); Lang, Laura Smith, Bedford, Texas 76021 (US)
(74) Representative: Reinhard - Skuhra - Weise & Partner

(56) References cited:
- EP-A- 0 013 376
- EP-A- 0 217 206
- EP-A- 0 217 206
- EP-A- 0 257 882
- FR-A- 2 313 031
- FR-A- 2 313 052
- SCAND. J. RHEUMATOLOGY, vol. 75, 1988, pages 290-299; R.R. BARTLETT ET AL.: 'DEVELOPMENT OF AUTOIMMUNITY IN MRL/lpr MICE AND THE EFFECTS OF DRUGS ON'
- R. BERKOW ET AL.: "Allergic conjunctivitis", 1987, THE MERCK MANUAL, MERCK & CO., INC., RAHWAY, N.J., US
- INT. J. IMMUNOPHARMACOL., vol. 8, no. 2, 1986, pages 199-204; R.R. BARTLETT: 'IMMUNOPHARMACOLOGICAL PROFILE OF HWA 486, A NOVEL ISOXAZOL DERIVATIVE'
- SURVEY OF OPHTHALMOLOGY, vol. 31, no. 3, 1986, pages 159-169; R.B. NUSSENBLATT ET AL.: 'CYCLOSPORINE: IMMUNOLOGY, PHARMACOLOGY AND THERAPEUTIC USES'

## Description

### Background of the Invention

This invention relates to the use of certain 5-methyl-isoxazole-4-carboxylic acid anilides and 2-hydroxyethylidene-cyano acetic acid anilides for the preparation of a pharmaceutical formulation in treating ocular diseases with immune etiology and to pharmaceutical formulations adapted for ophthalmic administration comprising a therapeutically effective amount of these compounds and uses thereof. The compounds are also useful to prolong graft survival of corneal or other ocular tissues and as surgical adjuncts in patients who are atopic or immune impaired.

The 5-methyl-isoxazole-4-carboxylic acid anilides are generically disclosed in U.S. Patent No. 4,087,535, which patent is fully incorporated herein by reference to the extent it defines the 5-methyl-isoxazole-4-carboxylic acid anilides and their synthesis. The compound, 5-methylisoxazole-4-carboxylic acid-4-trifluoromethyl-anilide (leflunomide) which is encompassed within the generic class, is specifically disclosed in U.S. Patent Nos. 4,284,786 and 4,351,841. The metabolite of leflunomide and the metabolite's derivatives are described in U.S. Patent No. 4,061,767 which is fully incorporated herein by reference to the extent it defines these compounds, which are 2-hydroxyethylidene-cyano acetic acid anilides, and their synthesis. Leflunomide's use as an antirheumatic, antiphlogistic, antipyretic, analgesic and as a compound for combating multiple sclerosis is also disclosed. In U.S. Patent Application No. 911,328, now U.S. Patent No. 4,965,276, leflunomide and its metabolite, herein referred to as AL 3318, are disclosed for combatting chronic graft-versus-host and autoimmune diseases. Ocular indications and topical administration is not discussed.

SCAND. J. RHEUMATOLOGY, vol. 75, 1988, p. 290 - 299, BARTLETT et. al, discloses some effects of HWA 486 on MRL/pr mice. Treating these mice with HWA 486 resulted in decreased autoantibodies, decreased immune complex deposits on the glomeruli, restoration of depressed immune response and extended longevity of the MRL/pr mice.

EP-A-0 217 206 discloses that the 4-trifluoromethylanilide of 5-methylisoxazole-4-carboxylic acid and its metabolite have immunomodulating properties such that they are suitable as medicaments to combat chronic graft-versus-host diseases and to combat autoimmune diseases, particularly systemic lupus erythematosus.

Steroids and antimetabolite compounds, such as cyclophosphamide, have been used orally to treat severe uveitis, such as that associated with Behcet's disease. Oral steroid therapy is usually accompanied by the topical use of steroid therapy (ocular) to more rapidly control the inflammation. Steroids are also used in conjunction with antiviral, antiparasitic or antifungal agents to treat uveitis associated with microbial infections. Both antimetabolite and steroid therapies are general immunosuppressive treatments with ocular and systemic side effects.

Cyclosporin A (CsA), a fungal-derived immunosuppressive agent, has recently been used to treat dry eye (in dogs), severe uveitis, vernal conjunctivitis and to prevent corneal graft rejection in humans; see, for example, Nussenblatt et al., Survey of Ophthalmology, Vol.31, No.3 (Nov.-Dec.,1986); and BenEzra et al., American Journal of Ophthalmology, Vol.101, p.298 (Mar.,1986). CsA is effective, but has major side effects, including kidney damage and predilection for tumor formation. This makes long term therapeutic use, which is usually necessary, deleterious. In addition, due to its size and structure, CsA is not water soluble and currently must be delivered in a suitable lipophilic formulation which is not optimal for topical ophthalmic use.

### Summary of the Invention

The present invention is directed to the use of a therapeutically effective amount of 5-methyl-isoxazole-4-carboxylic acid anilides and hydroxyethylidene-cyano acetic acid anilide derivatives for the preparation of a pharmaceutical formulation for treating ocular diseases with immune etiology, to a pharmaceutical formulation adapted for ophthalmic administration containing these compounds and uses thereof. In addition the compounds are useful for treating ocular manifestations associated with systemic diseases with immune etiology. The compounds exhibit immunosuppressive, antiinflammatory, and mild antiallergic activity and are useful for the treatment of eye diseases such as uveitis (including rheumatoid nodules), retinitis, allergy (vernal keratoconjunctivitis and allergic or giant papillary conjunctivitis) and dry eye (Sjogren's syndrome). Additionally the compounds are useful for prolonging graft survival of corneal or other ocular tissue and are useful as surgical adjuncts in patients which are atopic or immune impaired.

The compounds are not universally cytotoxic, thereby overcoming the antimetabolite toxicity problems associated with the use of, for example, cyclophosphamide. In addition, the problems associated with the long term use of steroids are not encountered. The compounds of the present invention offer an alternative to cyclosporin and the complications associated with its long term use.

The compounds can be used for the treatment of ocular diseases and ocular manifestations associated with systemic diseases with immune etiology via oral, intravenous, intramuscular, topical and/or intraocular administration.

### Detailed Description of Preferred Embodiments

The present invention relates to the use of a therapeutically effective amount of compounds with the following formulas: 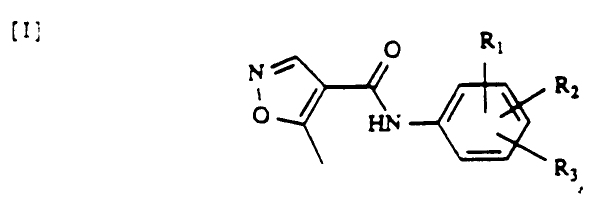 in which R₁, R₂ and R₃, which may be identical or different, each stand for an alkyl group of 1,2 or 3 carbon atoms, an alkoxy group of 1,2 or 3 carbon atoms, an alkylthio group of 1,2 or 3 carbon atoms, which groups may be substituted partly or totally by identical or different halogen atoms, such as fluorine, chlorine, bromine or iodine atoms;
halogen atoms, such as fluorine, chlorine, bromine or iodine;
nitro;
cyano;
alkoxycarbonyl groups of 1,2 or 3 carbon atoms in the alkyl moiety, and in which R₁ and R₂ each further stands for hydrogen, in which case, however, R₃ cannot stand for methyl but additionally can stand for a phenyl group which may carry one or two fluorine, chlorine, bromine or iodine atoms, alkyl groups of 1,2 or 3 carbon atoms or alkoxy groups of 1,2 or 3 carbon atoms, or for a phenoxy group which may carry one or two fluorine, chlorine, bromine or iodine atoms, alkyl groups of 1,2 or 3 carbon atoms or alkyoxy groups of 1,2 or 3 carbon atoms, or in which R₁ stands for hydrogen, and R₂ and R₃ together stand for a methylene-dioxy group or together with the phenyl ring, to which they are linked, they stand for a naphthalene ring; and 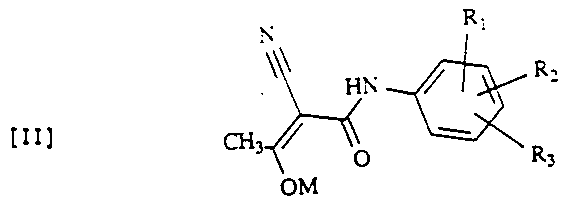 in which R¹, R² and R³, which may be identical or different, each stands for a halogen substituent, such as chlorine, fluorine, or bromine, an alkyl group of 1, 2, 3 or 4 carbon atoms, an alkoxy group of 1, 2 or 3 carbon atoms, an alkylthio group of 1, 2 or 3 carbon atoms, which groups may be substituted entirely or partly by identical or different halogen atoms, such as fluorine, chlorine, bromine or iodine; for a nitro, cyano or alkoxycarbonyl group of 1, 2 or 3 carbon atoms in the alkyl moiety; R¹ and R² each further stands for hydrogen, in which case, however, R³ cannot stand for methyl but additionally stands for a phenyl group which may carry one or two fluorine, chlorine, bromine or iodine atoms, alkyl groups of 1, 2 3 or 4 carbon atoms, or alkoxy groups of 1, 2 or 3 carbon atoms, or for a phenoxy group which may carry one or two fluorine, chlorine, bromine or iodine atoms, alkyl groups of 1, 2 or 3 carbon atoms or alkoxy groups of 1, 2 or 3 carbon atoms, or R¹ stands for hydrogen, and R² and R³ together stand for a methylene-dioxy group, or together with the phenyl ring, to which they are linked, they stand for a naphthalene ring, and in which M stands for hydrogen, an alkali metal, such as sodium or potassium, or ammonium and a pharmaceutically acceptable carrier for the preparation of a pharmaceutical formulation for treating ocular diseases with immune etiology.

The compounds described in [I] and [II] above exhibit antiallergic, antiinflammatory and/or immunomodulating activity. Because it is believed that the compounds are not general or broad spectrum immunosuppressants, such as antimetabolites and steroids, and because it is believed the compounds suppress T and B cell functions differently than CsA, the compounds of the present invention offer an alternative method for the treatment of ocular diseases and ocular manifestations of systemic diseases with immune etiology, collectively referred to herein as "ocular diseases".

The compounds represented by I and II above may be administered orally, intravenously, intramuscularly, topically and/or intraocularly for the treatment of ocular diseases. The compounds may be formulated as tablets, solutions or suspensions. The dose may vary from about 0.0001 mg/kg/day to about 30 mg/kg/day. Topical or intraocular delivery can include the use of ointments, liposomes, microspheres, palmitic-acid attachment or other lipid-like molecules useful in enhancing delivery to the eye. Oil or oil-like vehicles may also be used, however, an aqueous based vehicle is preferred.

The present invention relates, furthermore, to a pharmaceutical formulation adapted for ophthalmic administration characterized by comprising:
(a) a therapeutically effective amount of a compound with the formula: 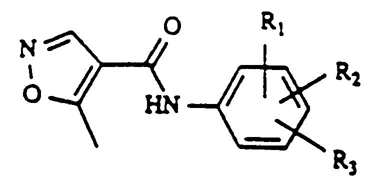 wherein: R₁, R₂ and R₃, which may be identical or different, each stand for an alkyl group of 1, 2 or 3 carbon atoms, an alkoxy group of 1, 2 or 3 carbon atoms, an alkylthio group of 1, 2 or 3 carbon atoms, which groups may be substituted partly or totally by identical or different halogen atoms, such as fluorine, chlorine, bromine or iodine atoms;
   halogen atoms, such as fluorine, chlorine, bromine or iodine;
   nitro;
   cyano;
   alkoxycarbonyl groups of 1, 2 or 3 carbon atoms in the alkyl moiety, and in which R₁ and R₂ each further stands for hydrogen, in which case, however, R₃ cannot stand for methyl but additionally can stand for a phenyl group which may carry one or two fluorine, chlorine, bromine or iodine atoms, alkyl groups of 1, 2 or 3 carbon atoms or alkoxy groups of 1, 2 or 3 carbon atoms, or for a phenoxy group which may carry one or two fluorine, chlorine, bromine or iodine atoms, alkyl groups of 1, 2 or 3 carbon atoms or alkyoxy groups of 1, 2 or 3 carbon atoms, or in which R₁ stands for hydrogen, R₂ and R₃ together stand for a methylene-dioxy group or together with the phenyl ring, to which they are linked, they stand for a naphthalene ring; and
(b) a pharmaceutically acceptable carrier.

Furthermore, the present invention relates to a pharmaceutical formulation adapted for ophthalmic administration comprising
(a) a therapeutically effective amount of a metabolite compound, said metabolite being of the formula: 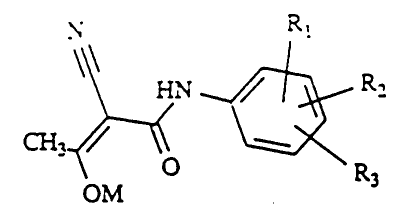 in which R₁, R₂ and R₃, which may be identical or different, each stand for a halogen, such as chlorine, fluorine or bromine, an alkyl group of 1, 2, 3 or 4 carbon atoms, an alkoxy group of 1, 2 or 3 carbon atoms, an alkylthio group of 1, 2 or 3 carbon atoms, which groups may be substituted entirely or partly by identical or different halogen atoms, such as fluorine, chlorine, bromine or iodine; for a nitro, cyano or alkoxycarbonyl group of 1, 2 or 3 carbon atoms in the alkyl moiety; R₁ and R₂ each further stands for hydrogen, in which case, however, R₃ cannot stand for methyl but additionally stands for a phenyl group which may carry one or two fluorine, chlorine, bromine or iodine atoms, alkyl groups of 1, 2, 3 or 4 carbon atoms, or alkoxy groups of 1, 2 or 3 carbon atoms, or for a phenoxy group which may carry one or two fluorine, chlorine, bromine or iodine atoms, alkyl groups of 1, 2 or 3 carbon atoms or alkyoxy groups of 1, 2 or 3 carbon atoms, or R₁ stands for hydrogen and R₂ and R₃ together stand for a methylene-dioxy group, or together with the phenyl ring, to which they are linked, they stand for a naphthalene ring, and in which M stands for hydrogen, an alkali metal, such as sodium or potassium, or ammonium; and
(b) a pharmaceutically acceptable carrier.

Furthermore, the present invention relates to uses thereof.

The preferred compounds of the present invention have the following structures: 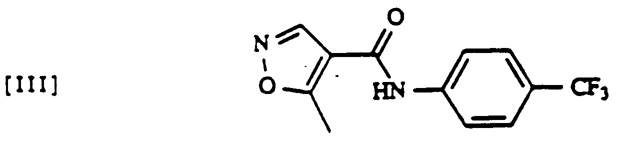 N-(4-Trifluoro-methylphenyl)-5-methylisoxazole-4-carboxamide (Leflunomide) 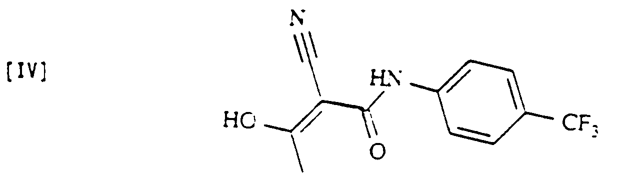 N-(4-trifluoromethylphenyl)-2-cyano-3-hydroxycrotonamide (AL 3318)

Additional compounds which can be used according to the present invention based on structure II include: 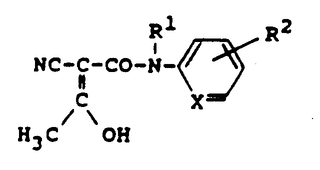 wherein:

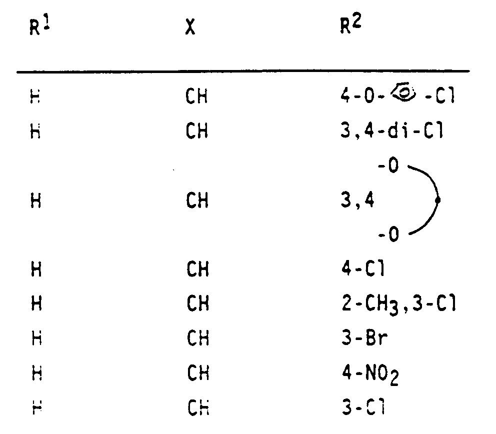

For the treatment of uveitis, dry eye and conjunctivitis leflunomide or AL 3318 can be administered systemically at a concentration of about 0.01-10 mg/kg/day or topically four times daily at a concentration of about 0.05-10 percent by weight (wt.%).

For the prevention of corneal graft rejection leflunomide or AL 3318 can be administered topically at about 0.05-20 wt.%; or systemically at about 0.01-30 mg/kg/day.

The following Examples illustrate formulations of the compounds of the present invention useful for the topical treatment of ocular diseases, particularly uveitis. They are in no way limiting.

| EXAMPLE 1 (Suspension) | |
|---|---|
| Ingredient | Concentrations(wt.%) |
| Hydroxypropyl methylcellulose (HPMC) | 0.5 |
| Na2HPO₄ | 0.2 |
| NaCl | 0.8 |
| EDTA | 0.01 |
| Benzalkonium chloride (BAC) | 0.01 |
| Tween 80 | 0.05 |
| Purified water | q.s. volume |
| pH | 7.4 |
| Osmolality | 290 mOsm/kg |
| Leflunomide | 1.0 + 2% xs |

### Procedure

10ml of the above suspension was made by first making the vehicle. This was done by adding, and quickly stirring, 2.508g of HPMC in 250ml of water at 90-100^{o}C. The composition was cooled to 5^{o}C with stirring. 1.013g Na₂HPO₄, 4.002g NaCl, 0.0535g EDTA, 5.002g BAC, 0.2569g Tween were dissolved in about 200ml of water, filtered through a corning 0.2 um filter unit and then added to the HPMC composition. The resulting mixture was brought to 500ml with water. A suspension of leflunomide was then prepared by ball milling 0.1006g leflunomide and 10ml of the mixture made above with about 3g of glass beads for 2 hours. The final suspension was then brought to volume with the mixture made above.

| EXAMPLE 2 (Aqueous) | |
|---|---|
| Ingredient | Concentration(wt.%) |
| Na2HPO₄ | 0.2 |
| NaCl | 0.8 |
| EDTA | 0.01 |
| BAC | 0.01 |
| Tween 80 | 0.05 |
| Purified water | q.s. |
| pH | 7.42 |
| Osmolality | 291 mOsm/kg |
| Leflunomide | 1.0 |

### Preparation

The above formulation can be made according to procedures known to those skilled in the art of making pharmaceutical preparations.

| EXAMPLE 3 (Ointment) | |
|---|---|
| Ingredient | Concentration(wt.%) |
| Leflunomide | 1.0 |
| Methyl paraben | 0.05 |
| Propyl paraben | 0.01 |
| Anhydrous liquid lanolin | 4.0 |
| Mineral oil | 15.0 |
| White petrolatum | 79.94 |

### Procedure

The above formulation can be made according to procedures known to those skilled in the art of making pharmaceutical preparations.

| EXAMPLE 4 (Suspension) | |
|---|---|
| Ingredient | Concentration(wt.%) |
| Leflunomide | 0.5 + 2% xs |
| Carbopol 934P | 0.5 |
| Sodium chloride, USP | 0.4 |
| Mannitol, USP | 2.0 |
| Polysorbate 80, NF | 0.25 |
| Benzalkonium Chloride, NF | 0.01 + 5% xs |
| Disodium Edetate, USP | 0.01 |
| Hydrochloric acid, NF and/or sodium hydroxide, NF | |
| pH | 7.2 |
| Water for injection, USP | q.s. volume |

### Procedure

The above suspension was prepared by first making the vehicle. The 500ml of vehicle was prepared by adding to a 600ml beaker, 10.0095g of mannitol, 2.0002g of NaCl, 0.0578g EDTA and 2.510g of carbopol to 400g of water and stirring until homogenous. The pH was adjusted to 7 ⁺ 0.1 with NaOH. In a 50ml beaker 0.1018g BAC, and 1.2616g Tween were added to about 30ml of water and stirred, and added to the carbopol mixture, and water added to 500g. A separate suspension of 0.1277g leflunomide and 25.14g of the above described vehicle was made. 20g of that suspension and 10g of 4mm glass beads were stirred for 2 days.

## Claims (Claims for the following Contracting State(s): AT, BE, DE, DK, FR, GB, IT, LI, LU, NL, SE)

1. Use of
(a) a therapeutically effective amount of a compound with the formula 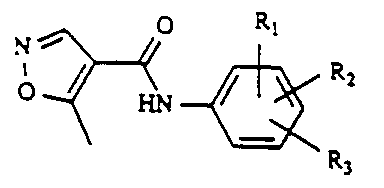 wherein: R₁, R₂ and R₃, which may be identical or different, each stand for an alkyl group of 1, 2 or 3 carbon atoms, an alkoxy group of 1, 2 or 3 carbon atoms, an alkylthio group of 1, 2 or 3 carbon atoms, which groups may be substituted partly or totally by identical or different halogen atoms, such as fluorine, chlorine, bromine or iodine atoms;
halogen atoms, such as fluorine, chlorine, bromine or iodine;
nitro;
cyano;
alkoxycarbonyl groups of 1, 2 or 3 carbon atoms in the alkyl moiety, and in which R₁ and R₂ each further stands for hydrogen, in which case, however, R₃ cannot stand for methyl but additionally can stand for a phenyl group which may carry one or two fluorine, chlorine, bromine or iodine atoms, alkyl groups of 1, 2 or 3 carbon atoms or alkoxy groups of 1, 2 or 3 carbon atoms, or for a phenoxy group which may carry one or two fluorine, chlorine, bromine or iodine atoms, alkyl groups of 1, 2 or 3 carbon atoms or alkyoxy groups of 1, 2 or 3 carbon atoms, or in which R₁ stands for hydrogen, R₂ and R₃ together stand for a methylene-dioxy group or together with the phenyl ring, to which they are linked, they stand for a naphthalene ring; and
(b) a pharmaceutically acceptable carrier for the preparation of a pharmaceutical formulation for treating ocular diseases with immune etiology.

2. Use of a therapeutically effective amount of a compound of the formula: 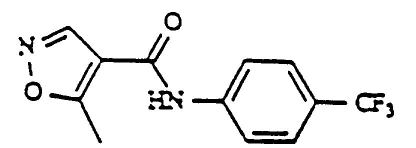 for the preparation of a pharmaceutical formulation for treating ocular diseases with immune etiology according to claim 1.

3. Use of
(a) a therapeutically effective amount of a compound of the formula 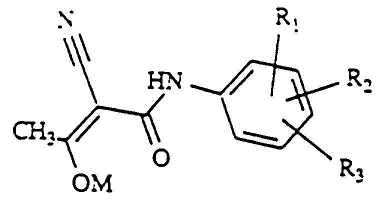 in which R₁, R₂ and R₃, which may be identical or different, each stand for a halogen, such as chlorine, fluorine or bromine, an alkyl group of 1, 2, 3 or 4 carbon atoms, an alkoxy group of 1, 2 or 3 carbon atoms, an alkylthio group of 1, 2 or 3 carbon atoms, which groups may be substituted entirely or partly by identical or different halogen atoms, such as fluorine, chlorine, bromine or iodine; for a nitro, cyano or alkoxycarbonyl group of 1, 2 or 3 carbon atoms in the alkyl moiety; R₁ and R₂ each further stands for hydrogen, in which case, however, R₃ cannot stand for methyl but additionally stands for a phenyl group which may carry one or two fluorine, chlorine, bromine or iodine atoms, alkyl groups of 1, 2, 3 or 4 carbon atoms, or alkoxy groups of 1, 2 or 3 carbon atoms, or for a phenoxy group which may carry one or two fluorine, chlorine, bromine or iodine atoms, alkyl groups of 1, 2 or 3 carbon atoms or alkyoxy groups of 1, 2 or 3 carbon atoms, or R₁ stands for hydrogen and R₂ and R₃ together stand for a methylene-dioxy group, or together with the phenyl ring, to which they are linked, they stand for a naphthalene ring, and in which M stands for hydrogen, an alkali metal, such as sodium or potassium, or ammonium; and
(b) a pharmaceutically acceptable carrier for the preparation of a pharmaceutical formulation for treating ocular diseases with immune etiology according to claim 1.

4. Use of a therapeutically effective amount of a compound of the formula 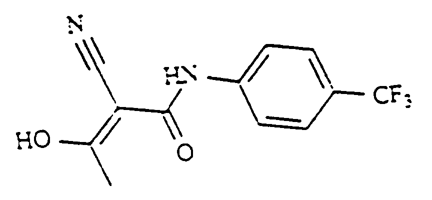 for the preparation of a pharmaceutical formulation for treating ocular diseases with immune etiology according to claim 3.

5. Use of a therapeutically effective amount of a compound of the formula: 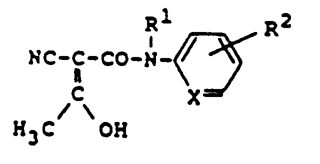 wherein: 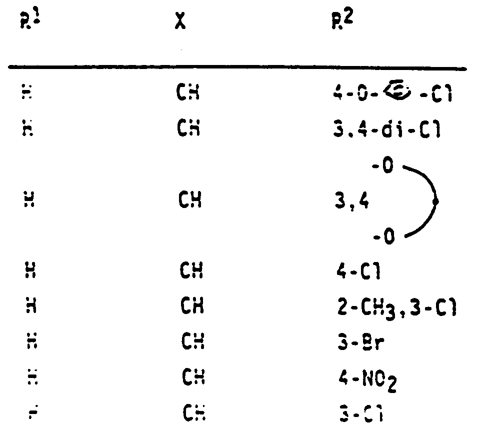 for the preparation of a pharmaceutical formulation for treating ocular diseases with immune etiology according to claim 3.

6. Use according to one or more of claims 1-5 characterized in that the compound is delivered at a concentration of between 0.0001-30 mg/kg/day, preferably at a concentration of 0.01 to 10.0 mg/kg/day.

7. Use according to one or more of claims 1-6 characterized in that the ocular disease with immune etiology is selected from the group consisting of uveitis, retinitis, allergy, and dry eye.

8. Use according to one or more of claims 1-6 further being useful for prolonging graft survival of corneal or other ocular tissue and as surgical adjuncts in patients which are atopic or immune impaired.

9. A pharmaceutical formulation adapted for ophthalmic administration characterized by comprising:
(a) a therapeutically effective amount of a compound with the formula: 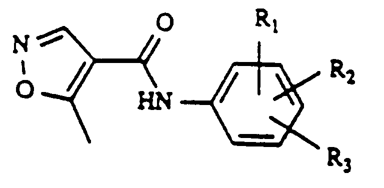 wherein: R₁, R₂ and R₃, which may be identical or different, each stand for an alkyl group of 1, 2 or 3 carbon atoms, an alkoxy group of 1, 2 or 3 carbon atoms, an alkylthio group of 1, 2 or 3 carbon atoms, which groups may be substituted partly or totally by identical or different halogen atoms, such as fluorine, chlorine, bromine or iodine atoms;
halogen atoms, such as fluorine, chlorine, bromine or iodine;
nitro;
cyano;
alkoxycarbonyl groups of 1, 2 or 3 carbon atoms in the alkyl moiety, and in which R₁ and R₂ each further stands for hydrogen, in which case, however, R₃ cannot stand for methyl but additionally can stand for a phenyl group which may carry one or two fluorine, chlorine, bromine or iodine atoms, alkyl groups of 1, 2 or 3 carbon atoms or alkoxy groups of 1, 2 or 3 carbon atoms, or for a phenoxy group which may carry one or two fluorine, chlorine, bromine or iodine atoms, alkyl groups of 1, 2 or 3 carbon atoms or alkyoxy groups of 1, 2 or 3 carbon atoms, or in which R₁ stands for hydrogen, R₂ and R₃ together stand for a methylene-dioxy group or together with the phenyl ring, to which they are linked, they stand for a naphthalene ring; and
(b) a pharmaceutically acceptable carrier.

10. The formulation of claim 9 characterized in that the compound is a therapeutically effective amount of a compound of the formula

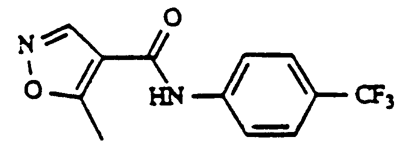

11. A pharmaceutical formulation according to claim 9 characterized by comprising:
(a) a therapeutically effective amount of a metabolite of a compound of claim 9, said metabolite being of the formula: 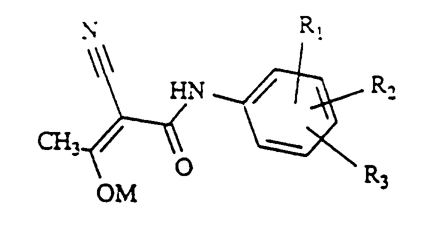 in which R₁, R₂ and R₃, which may be identical or different, each stand for a halogen, such as chlorine, fluorine or bromine, an alkyl group of 1, 2, 3 or 4 carbon atoms, an alkoxy group of 1, 2 or 3 carbon atoms, an alkylthio group of 1, 2 or 3 carbon atoms, which groups may be substituted entirely or partly by identical or different halogen atoms, such as fluorine, chlorine, bromine or iodine; for a nitro, cyano or alkoxycarbonyl group of 1, 2 or 3 carbon atoms in the alkyl moiety; R₁ and R₂ each further stands for hydrogen, in which case, however, R₃ cannot stand for methyl but additionally stands for a phenyl group which may carry one or two fluorine, chlorine, bromine or iodine atoms, alkyl groups of 1, 2, 3 or 4 carbon atoms, or alkoxy groups of 1, 2 or 3 carbon atoms, or for a phenoxy group which may carry one or two fluorine, chlorine, bromine or iodine atoms, alkyl groups of 1, 2 or 3 carbon atoms or alkyoxy groups of 1, 2 or 3 carbon atoms, or R₁ stands for hydrogen and R₂ and R₃ together stand for a methylene-dioxy group, or together with the phenyl ring, to which they are linked, they stand for a naphthalene ring, and in which M stands for hydrogen, an alkali metal, such as sodium or potassium, or ammonium; and
(b) a pharmaceutically acceptable carrier.

12. The formulation according to claim 11, characterized in that the metabolite is of the formula

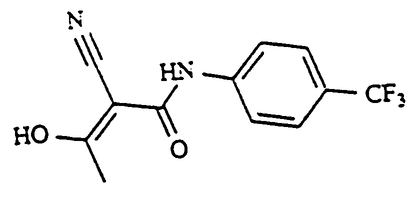

13. A pharmaceutical formulation according to claim 11,
characterized in that the compound is a therapeutically effective amount of a compound of the formula 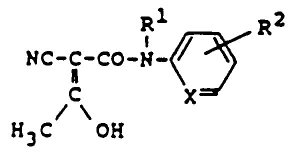 wherein:

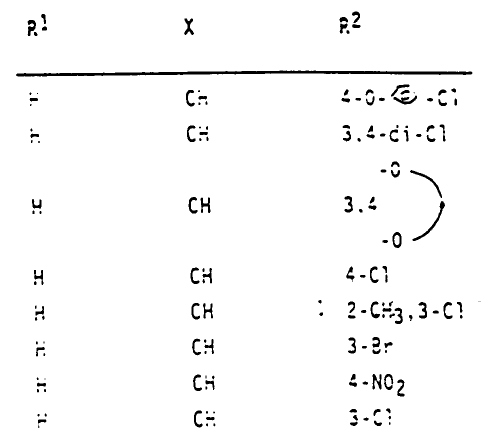

14. Use of
(a) a therapeutically effective amount of a compound with the formula 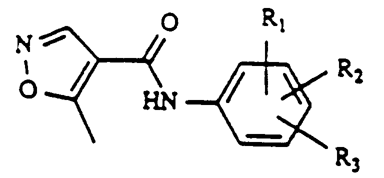 wherein: R₁, R₂ and R₃, which may be identical or different, each stand for an alkyl group of 1, 2 or 3 carbon atoms, an alkoxy group of 1, 2 or 3 carbon atoms, an alkylthio group of 1, 2 or 3 carbon atoms, which groups may be substituted partly or totally by identical or different halogen atoms, such as fluorine, chlorine, bromine or iodine atoms;
halogen atoms, such as fluorine, chlorine, bromine or iodine;
nitro;
cyano;
alkoxycarbonyl groups of 1, 2 or 3 carbon atoms in the alkyl moiety, and in which R₁ and R₂ each further stands for hydrogen, in which case, however, R₃ cannot stand for methyl but additionally can stand for a phenyl group which may carry one or two fluorine, chlorine, bromine or iodine atoms, alkyl groups of 1, 2 or 3 carbon atoms or alkoxy groups of 1, 2 or 3 carbon atoms, or for a phenoxy group which may carry one or two fluorine, chlorine, bromine or iodine atoms, alkyl groups of 1, 2 or 3 carbon atoms or alkyoxy groups of 1, 2 or 3 carbon atoms, or in which R₁ stands for hydrogen, R₂ and R₃ together stand for a methylene-dioxy group or together with the phenyl ring, to which they are linked, they stand for a naphthalene ring; and
(b) a pharmaceutically acceptable carrier for the preparation of a pharmaceutical formulation adapted for ophthalmic administration for treating ocular diseases with immune etiology.

15. Use of a therapeutically effective amount of a compound of the formula: 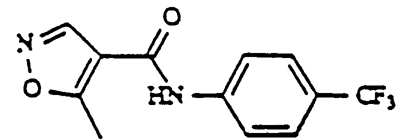 for the preparation of a pharmaceutical formulation adapted for ophthalmic administration for treating ocular diseases with immune etiology according to claim 14.

16. Use of
(a) a therapeutically effective amount of a compound of the formula 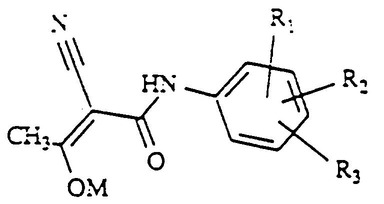 in which R₁, R₂ and R₃, which may be identical or different, each stand for a halogen, such as chlorine, fluorine or bromine, an alkyl group of 1, 2, 3 or 4 carbon atoms, an alkoxy group of 1, 2 or 3 carbon atoms, an alkylthio group of 1, 2 or 3 carbon atoms, which groups may be substituted entirely or partly by identical or different halogen atoms, such as fluorine, chlorine, bromine or iodine; for a nitro, cyano or alkoxycarbonyl group of 1, 2 or 3 carbon atoms in the alkyl moiety; R₁ and R₂ each further stands for hydrogen, in which case, however, R₃ cannot stand for methyl but additionally stands for a phenyl group which may carry one or two fluorine, chlorine, bromine or iodine atoms, alkyl groups of 1, 2, 3 or 4 carbon atoms, or alkoxy groups of 1, 2 or 3 carbon atoms, or for a phenoxy group which may carry one or two fluorine, chlorine, bromine or iodine atoms, alkyl groups of 1, 2 or 3 carbon atoms or alkyoxy groups of 1, 2 or 3 carbon atoms, or R₁ stands for hydrogen and R₂ and R₃ together stand for a methylene-dioxy group, or together with the phenyl ring, to which they are linked, they stand for a naphthalene ring, and in which M stands for hydrogen, an alkali metal, such as sodium or potassium, or ammonium; and
(b) a pharmaceutically acceptable carrier for the preparation of a pharmaceutical formulation adapted for ophthalmic administration for treating ocular diseases with immune etiology according to claim 14.

17. Use of a therapeutically effective amount of a compound of the formula 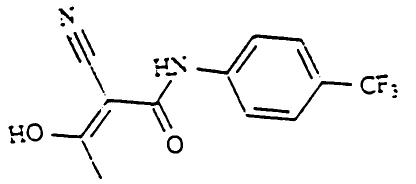 for the preparation of a pharmaceutical formulation adapted for ophthalmic administration for treating ocular diseases with immune etiology according to claim 16.

18. Use of a therapeutically effective amount of a compound of the formula: 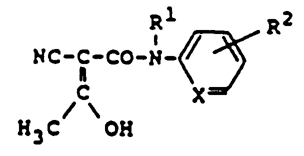 wherein: 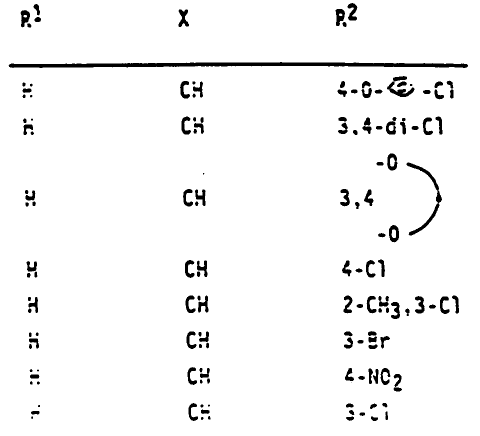 for the preparation of a pharmaceutical formulation adapted for ophthalmic administration for treating ocular diseases with immune etiology according to claim 16.

19. Use according to one or more of claims 14-18 characterized in that the compound is delivered at a concentration of between 0.0001-30 mg/kg/day, preferably at a concentration of 0.01 to 10.0 mg/kg/day.

20. Use according to one or more of claims 14-18 characterized in that the compound is delivered topically to the eye at a concentration of 0.05 to 10.0 wt.%.

21. Use according to one or more of claims 14-18 characterized in that the ocular disease with immune etiology is selected from the group consisting of uveitis, retinitis, allergy, and dry eye.

22. Use according to one or more of claims 14-18 further being useful for prolonging graft survival of corneal or other ocular tissue and as surgical adjuncts in patients which are atopic or immune impaired.

## Claims (Claims for the following Contracting State(s): ES)

1. Use of
(a) a therapeutically effective amount of a compound with the formula 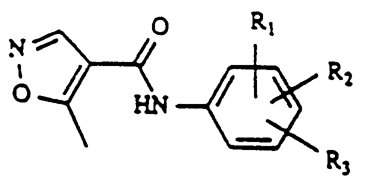 wherein: R₁, R₂ and R₃, which may be identical or different, each stand for an alkyl group of 1, 2 or 3 carbon atoms, an alkoxy group of 1, 2 or 3 carbon atoms, an alkylthio group of 1, 2 or 3 carbon atoms, which groups may be substituted partly or totally by identical or different halogen atoms, such as fluorine, chlorine, bromine or iodine atoms;
halogen atoms, such as fluorine, chlorine, bromine or iodine;
nitro;
cyano;
alkoxycarbonyl groups of 1, 2 or 3 carbon atoms in the alkyl moiety, and in which R₁ and R₂ each further stands for hydrogen, in which case, however, R₃ cannot stand for methyl but additionally can stand for a phenyl group which may carry one or two fluorine, chlorine, bromine or iodine atoms, alkyl groups of 1, 2 or 3 carbon atoms or alkoxy groups of 1, 2 or 3 carbon atoms, or for a phenoxy group which may carry one or two fluorine, chlorine, bromine or iodine atoms, alkyl groups of 1, 2 or 3 carbon atoms or alkyoxy groups of 1, 2 or 3 carbon atoms, or in which R₁ stands for hydrogen, R₂ and R₃ together stand for a methylene-dioxy group or together with the phenyl ring, to which they are linked, they stand for a naphthalene ring; and
(b) a pharmaceutically acceptable carrier for the preparation of a pharmaceutical formulation for treating ocular diseases with immune etiology.

2. Use of a therapeutically effective amount of a compound of the formula: 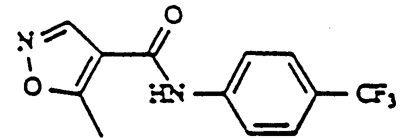 for the preparation of a pharmaceutical formulation for treating ocular diseases with immune etiology according to claim 1.

3. Use of
(a) a therapeutically effective amount of a compound of the formula 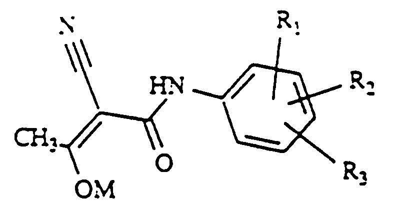 in which R₁, R₂ and R₃, which may be identical or different, each stand for a halogen, such as chlorine, fluorine or bromine, an alkyl group of 1, 2, 3 or 4 carbon atoms, an alkoxy group of 1, 2 or 3 carbon atoms, an alkylthio group of 1, 2 or 3 carbon atoms, which groups may be substituted entirely or partly by identical or different halogen atoms, such as fluorine, chlorine, bromine or iodine; for a nitro, cyano or alkoxycarbonyl group of 1, 2 or 3 carbon atoms in the alkyl moiety; R₁ and R₂ each further stands for hydrogen, in which case, however, R₃ cannot stand for methyl but additionally stands for a phenyl group which may carry one or two fluorine, chlorine, bromine or iodine atoms, alkyl groups of 1, 2, 3 or 4 carbon atoms, or alkoxy groups of 1, 2 or 3 carbon atoms, or for a phenoxy group which may carry one or two fluorine, chlorine, bromine or iodine atoms, alkyl groups of 1, 2 or 3 carbon atoms or alkyoxy groups of 1, 2 or 3 carbon atoms, or R₁ stands for hydrogen and R₂ and R₃ together stand for a methylene-dioxy group, or together with the phenyl ring, to which they are linked, they stand for a naphthalene ring, and in which M stands for hydrogen, an alkali metal, such as sodium or potassium, or ammonium; and
(b) a pharmaceutically acceptable carrier for the preparation of a pharmaceutical formulation for treating ocular diseases with immune etiology according to claim 1.

4. Use of a therapeutically effective amount of a compound of the formula 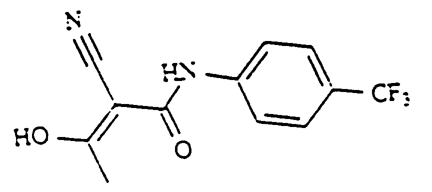 for the preparation of a pharmaceutical formulation for treating ocular diseases with immune etiology according to claim 3.

5. Use of a therapeutically effective amount of a compound of the formula: 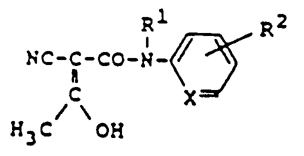 wherein: 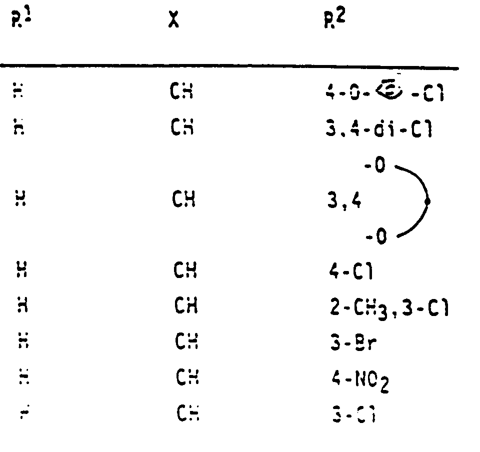 for the preparation of a pharmaceutical formulation for treating ocular diseases with immune etiology according to claim 3.

6. Use according to one or more of claims 1-5 characterized in that the compound is delivered at a concentration of between 0.0001-30 mg/kg/day, preferably at a concentration of 0.01 to 10.0 mg/kg/day.

7. Use according to one or more of claims 1-6 characterized in that the ocular disease with immune etiology is selected from the group consisting of uveitis, retinitis, allergy, and dry eye.

8. Use according to one or more of claims 1-6 further being useful for prolonging graft survival of corneal or other ocular tissue and as surgical adjuncts in patients which are atopic or immune impaired.

9. A process for the preparation of a pharmaceutical formulation comprising compounding
(a) a therapeutically effective amount of a compound with the formula: 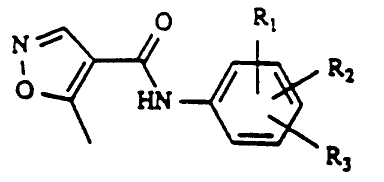 wherein: R₁, R₂ and R₃, which may be identical or different, each stand for an alkyl group of 1, 2 or 3 carbon atoms, an alkoxy group of 1, 2 or 3 carbon atoms, an alkylthio group of 1, 2 or 3 carbon atoms, which groups may be substituted partly or totally by identical or different halogen atoms, such as fluorine, chlorine, bromine or iodine atoms;
halogen atoms, such as fluorine, chlorine, bromine or iodine;
nitro;
cyano;
alkoxycarbonyl groups of 1, 2 or 3 carbon atoms in the alkyl moiety, and in which R₁ and R₂ each further stands for hydrogen, in which case, however, R₃ cannot stand for methyl but additionally can stand for a phenyl group which may carry one or two fluorine, chlorine, bromine or iodine atoms, alkyl groups of 1, 2 or 3 carbon atoms or alkoxy groups of 1, 2 or 3 carbon atoms, or for a phenoxy group which may carry one or two fluorine, chlorine, bromine or iodine atoms, alkyl groups of 1, 2 or 3 carbon atoms or alkyoxy groups of 1, 2 or 3 carbon atoms, or in which R₁ stands for hydrogen, R₂ and R₃ together stand for a methylene-dioxy group or together with the phenyl ring, to which they are linked, they stand for a naphthalene ring; and
(b) a pharmaceutically acceptable carrier to obtain a pharmaceutical formulation adapted for ophthalmic administration.

10. The process of claim 9 characterized in that the compound is a therapeutically effective amount of a compound of the formula

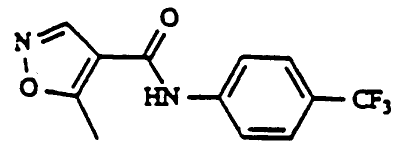

11. A process according to claim 9 characterized by comprising compounding
(a) a therapeutically effective amount of a metabolite of a compound of claim 9, said metabolite being of the formula: 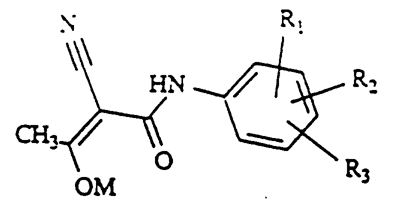 in which R₁, R₂ and R₃, which may be identical or different, each stand for a halogen, such as chlorine, fluorine or bromine, an alkyl group of 1, 2, 3 or 4 carbon atoms, an alkoxy group of 1, 2 or 3 carbon atoms, an alkylthio group of 1, 2 or 3 carbon atoms, which groups may be substituted entirely or partly by identical or different halogen atoms, such as fluorine, chlorine, bromine or iodine; for a nitro, cyano or alkoxycarbonyl group of 1, 2 or 3 carbon atoms in the alkyl moiety; R₁ and R₂ each further stands for hydrogen, in which case, however, R₃ cannot stand for methyl but additionally stands for a phenyl group which may carry one or two fluorine, chlorine, bromine or iodine atoms, alkyl groups of 1, 2, 3 or 4 carbon atoms, or alkoxy groups of 1, 2 or 3 carbon atoms, or for a phenoxy group which may carry one or two fluorine, chlorine, bromine or iodine atoms, alkyl groups of 1, 2 or 3 carbon atoms or alkyoxy groups of 1, 2 or 3 carbon atoms, or R₁ stands for hydrogen and R₂ and R₃ together stand for a methylene-dioxy group, or together with the phenyl ring, to which they are linked, they stand for a naphthalene ring, and in which M stands for hydrogen, an alkali metal, such as sodium or potassium, or ammonium; and
(b) a pharmaceutically acceptable carrier to obtain a pharmaceutical formulation adapted for ophthalmic administration.

12. The process according to claim 11, characterized in that the metabolite is of the formula

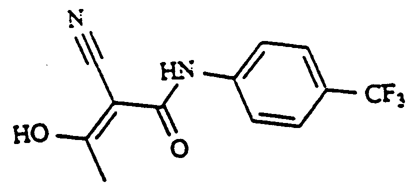

13. The process according to claim 11,
characterized in that the compound is a therapeutically effective amount of a compound of the formula 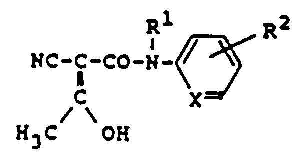 wherein:

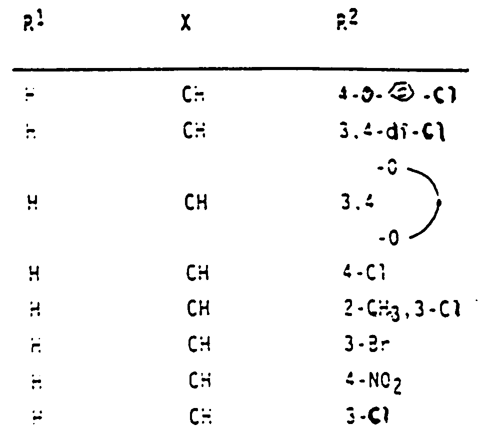

14. Use of
(a) a therapeutically effective amount of a compound with the formula 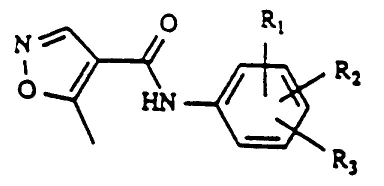 wherein: R₁, R₂ and R₃, which may be identical or different, each stand for an alkyl group of 1, 2 or 3 carbon atoms, an alkoxy group of 1, 2 or 3 carbon atoms, an alkylthio group of 1, 2 or 3 carbon atoms, which groups may be substituted partly or totally by identical or different halogen atoms, such as fluorine, chlorine, bromine or iodine atoms;
halogen atoms, such as fluorine, chlorine, bromine or iodine;
nitro;
cyano;
alkoxycarbonyl groups of 1, 2 or 3 carbon atoms in the alkyl moiety, and in which R₁ and R₂ each further stands for hydrogen, in which case, however, R₃ cannot stand for methyl but additionally can stand for a phenyl group which may carry one or two fluorine, chlorine, bromine or iodine atoms, alkyl groups of 1, 2 or 3 carbon atoms or alkoxy groups of 1, 2 or 3 carbon atoms, or for a phenoxy group which may carry one or two fluorine, chlorine, bromine or iodine atoms, alkyl groups of 1, 2 or 3 carbon atoms or alkyoxy groups of 1, 2 or 3 carbon atoms, or in which R₁ stands for hydrogen, R₂ and R₃ together stand for a methylene-dioxy group or together with the phenyl ring, to which they are linked, they stand for a naphthalene ring; and
(b) a pharmaceutically acceptable carrier for the preparation of a pharmaceutical formulation adapted for ophthalmic administration for treating ocular diseases with immune etiology.

15. Use of a therapeutically effective amount of a compound of the formula: 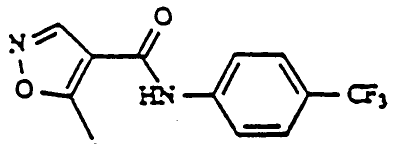 for the preparation of a pharmaceutical formulation adapted for ophthalmic administration for treating ocular diseases with immune etiology according to claim 14.

16. Use of
(a) a therapeutically effective amount of a compound of the formula 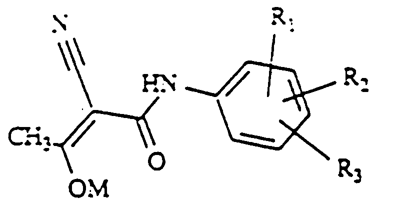 in which R₁, R₂ and R₃, which may be identical or different, each stand for a halogen, such as chlorine, fluorine or bromine, an alkyl group of 1, 2, 3 or 4 carbon atoms, an alkoxy group of 1, 2 or 3 carbon atoms, an alkylthio group of 1, 2 or 3 carbon atoms, which groups may be substituted entirely or partly by identical or different halogen atoms, such as fluorine, chlorine, bromine or iodine; for a nitro, cyano or alkoxycarbonyl group of 1, 2 or 3 carbon atoms in the alkyl moiety; R₁ and R₂ each further stands for hydrogen, in which case, however, R₃ cannot stand for methyl but additionally stands for a phenyl group which may carry one or two fluorine, chlorine, bromine or iodine atoms, alkyl groups of 1, 2, 3 or 4 carbon atoms, or alkoxy groups of 1, 2 or 3 carbon atoms, or for a phenoxy group which may carry one or two fluorine, chlorine, bromine or iodine atoms, alkyl groups of 1, 2 or 3 carbon atoms or alkyoxy groups of 1, 2 or 3 carbon atoms, or R₁ stands for hydrogen and R₂ and R₃ together stand for a methylene-dioxy group, or together with the phenyl ring, to which they are linked, they stand for a naphthalene ring, and in which M stands for hydrogen, an alkali metal, such as sodium or potassium, or ammonium; and
(b) a pharmaceutically acceptable carrier for the preparation of a pharmaceutical formulation adapted for ophthalmic administration for treating ocular diseases with immune etiology according to claim 14.

17. Use of a therapeutically effective amount of a compound of the formula 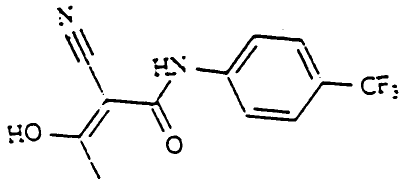 for the preparation of a pharmaceutical formulation adapted for ophthalmic administration for treating ocular diseases with immune etiology according to claim 16.

18. Use of a therapeutically effective amount of a compound of the formula: 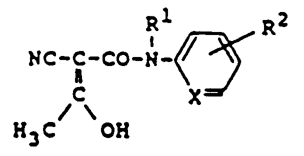 wherein: 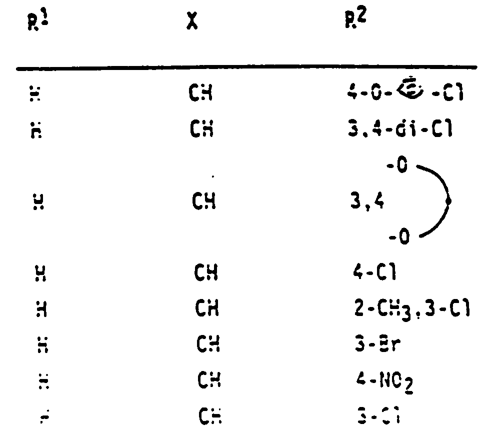 for the preparation of a pharmaceutical formulation adapted for ophthalmic administration for treating ocular diseases with immune etiology according to claim 16.

19. Use according to one or more of claims 14-18 characterized in that the compound is delivered at a concentration of between 0.0001-30 mg/kg/day, preferably at a concentration of 0.01 to 10.0 mg/kg/day.

20. Use according to one or more of claims 14-18 characterized in that the compound is delivered topically to the eye at a concentration of 0.05 to 10.0 wt.%.

21. Use according to one or more of claims 14-18 characterized in that the ocular disease with immune etiology is selected from the group consisting of uveitis, retinitis, allergy, and dry eye.

22. Use according to one or more of claims 14-18 further being useful for prolonging graft survival of corneal or other ocular tissue and as surgical adjuncts in patients which are atopic or immune impaired.

## Patentansprüche (Patentansprüche für folgende(n) Vertragsstaat(en): AT, BE, DE, DK, FR, GB, IT, LI, LU, NL, SE)

1. Verwendung
(a) einer therapeutisch wirksamen Menge einer Verbindung mit der Formel: 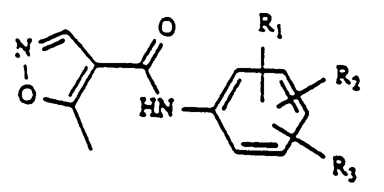 wobei R₁, R₂ und R₃, die gleich oder unterschiedlich sein können, je stehen für eine Alkylgruppe mit 1, 2 oder 3 Kohlenstoffatomen, eine Alkoxygruppe mit 1, 2 oder 3 Kohlenstoffatomen, eine Alkylthiogruppe mit 1, 2 oder 3 Kohlenstoffatomen, wobei die Gruppen teilweise oder vollständig durch identische oder unterschiedliche Halogenatome, wie Fluor-, Chlor-, Brom- oder Iodatome, substituiert sein können;
Halogenatome, wie Fluor, Chlor, Brom oder Iod;
Nitro;
Cyano;
Alkoxycarbonylgruppen mit 1, 2 oder 3 Kohlenstoffatomen im Alkylrest, und wobei R₁ und R₂ je weiterhin für Wasserstoff stehen, wobei in diesem Fall R₃ jedoch nicht Methyl bedeuten kann, sondern zusätzlich für eine Phenylgruppe stehen kann, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomen tragen kann, oder für eine Phenoxygruppe, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomen tragen kann, oder wobei R₁ für Wasserstoff steht, R₂ und R₃ zusammen für eine Methylen-Dioxy-Gruppe stehen oder sie zusammen mit dem Phenylring, an den sie gebunden sind, für einen Naphthalinring stehen; und
(b) eines pharmazeutisch verträglichen Trägers zur Herstellung einer pharmazeutischen Formulierung zur Behandlung von Augenerkrankungen mit Immunätiologie.

2. Verwendung einer therapeutisch wirksamen Menge einer Verbindung der Formel: 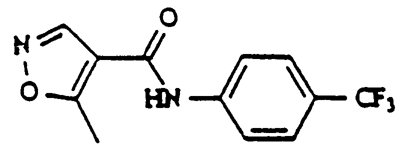 zur Herstellung einer pharmazeutischen Zubereitung zur Behandlung von Augenerkrankungen mit Immunätiologie nach Anspruch 1.

3. Verwendung
(a) einer therapeutisch wirksamen Menge einer Verbindung der Formel: 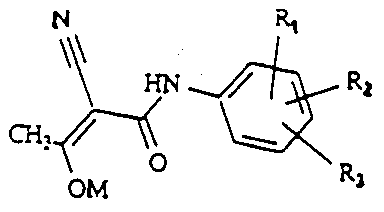 wobei R₁, R₂ und R₃, die gleich oder unterschiedlich sein können, je stehen für ein Halogen, wie Chlor, Fluor oder Brom, eine Alkylgruppe mit 1, 2, 3 oder 4 Kohlenstoffatomen, eine Alkoxygruppe mit 1, 2 oder 3 Kohlenstoffatomen, eine Alkylthiogruppe mit 1, 2 oder 3 Kohlenstoffatomen, wobei die Gruppen vollständig oder teilweise durch identische oder verschiedene Halogenatome, wie Fluor, Chlor, Brom oder Iod, substituiert sein können; für eine Nitro-, Cyano- oder Alkoxycarbonylgruppe mit 1, 2 oder 3 Kohlenstoffatomen im Alkylrest; R₁ und R₂ je weiterhin Wasserstoff bedeuten können, wobei in diesem Fall jedoch R₃ nicht Methyl bedeuten kann, sondern zusätzlich für eine Phenylgruppe steht, die 1 oder 2 Fluor-, Chlor-, Brom- oder Iodatome, Alkylgruppen mit 1, 2, 3 oder 4 Kohlenstoffatomen, oder Alkoxygruppen mit 1, 2 oder 3 Kohlenstoffatomen tragen kann, oder für eine Phenoxygruppe, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomen tragen kann oder R₁ für Wasserstoff steht und R₂ und R₃ zusammen für eine Methylen-Dioxy-Gruppe stehen, oder zusammen mit dem Phenylring, an den sie gebunden sind, für einen Naphthalinring stehen, und wobei M für Wasserstoff, ein Alkalimetall, wie Natrium oder Kalium, oder Ammonium steht; und
(b) eines pharmazeutisch verträglichen Trägers zur Herstellung einer pharmazeutischen Formulierung zur Behandlung von Augenerkrankungen mit Immunätiologie nach Anspruch 1.

4. Verwendung einer therapeutisch wirksamen Menge einer Verbindung der Formel: 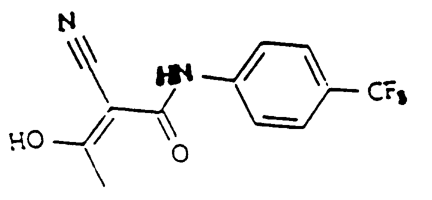 zur Herstellung einer pharmazeutischen Formulierung zur Behandlung von Augenerkrankungen mit Immunätiologie nach Anspruch 3.

5. Verwendung einer therapeutisch wirksamen Menge einer Verbindung der Formel: 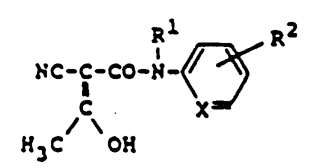 wobei 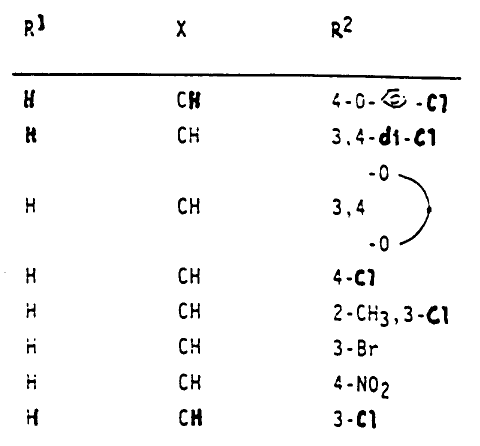 zur Herstellung einer pharmazeutischen Formulierung zur Behandlung von Augenerkrankungen mit Immunätiologie nach Anspruch 3.

6. Verwendung nach einem oder mehreren der Ansprüche 1-5, dadurch gekennzeichnet, daß die Verbindung in einer Konzentration von 0,0001-30 mg/kg/Tag, bevorzugt in einer Konzentration von 0,01-10,0 mg/kg/Tag, zugeführt wird.

7. Verwendung nach einem oder mehreren der Ansprüche 1-6, dadurch gekennzeichnet, daß die Augenerkrankung mit Immunätiologie zur Gruppe von Uveitis, Retinitis, Allergie und trockenem Auge ausgewählt wird.

8. Verwendung nach einem oder mehreren der Ansprüche 1-6, weiterhin einsetzbar zur Verlängerung der Beständigkeit von Transplantaten von Cornea- oder anderen Augengeweben und als chirurgische Zusätze bei Patienten, die atopisch sind oder an Störungen des Immunsystems leiden.

9. Pharmazeutische Formulierung, die zur ophthalmischen Verabreichung ausgelegt ist, dadurch gekennzeichnet, daß sie umfasst:
(a) eine therapeutisch wirksame Menge einer Verbindung mit der Formel: 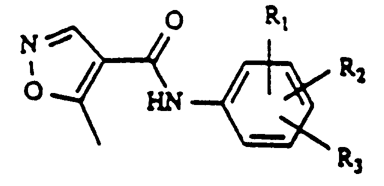 wobei R₁, R₂ und R₃, die gleich oder unterschiedlich sein können, je stehen für eine Alkylgruppe mit 1, 2 oder 3 Kohlenstoffatomen, eine Alkoxygruppe mit 1, 2 oder 3 Kohlenstoffatomen, eine Alkylthiogrupe mit 1, 2 oder 3 Kohlenstoffatomen, wobei die Gruppen teilweise oder vollständig durch identische oder unterschiedliche Halogenatome, wie Fluor-, Chlor-, Brom oder Iodatome, substituiert sein können;
Halogenatome, wie Fluor, Chlor, Brom oder Iod;
Nitro;
Cyano;
Alkoxycarbonylgruppen mit 1, 2 oder 3 Kohlenstoffatomen im Alkylrest, und wobei R₁ und R₂ je weiterhin für Wasserstoff stehen, wobei in diesem Fall R₃ jedoch nicht Methyl bedeuten kann, sondern zusätzlich für eine Phenylgruppe stehen kann, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomen tragen kann, oder für eine Phenoxygruppe, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomen tragen kann, oder wobei R₁ für Wasserstoff steht, R₂ und R₃ zusammen für ein eine Methylen-Dioxy-Gruppe stehen oder sie zusammen mit dem Phenylring, an den sie gebunden sind, für einen Naphthanlinring stehen; und
(b) einen pharmazeutisch verträglichen Träger.

10. Formulierung nach Anspruch 9, dadurch gekennzeichnet, daß die Verbindung eine therapeutisch wirksame Menge einer Verbindung der nachfolgenden Formel darstellt:

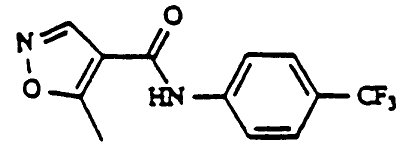

11. Pharmazeutische Formulierung nach Anspruch 9, dadurch gekennzeichnet, daß sie umfasst:
(a) eine therapeutisch wirksame Menge eines Metaboliten einer Verbindung des Anspruchs 9, wobei der Metabolit nachfolgende Formel aufweist: 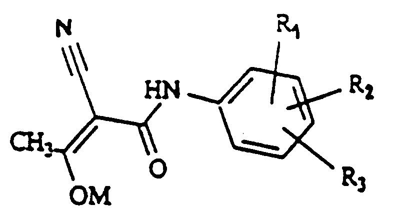 wobei R₁, R₂ und R₃, die gleich oder unterschiedlich sein können, je stehen für ein Halogen, wie Chlor, Fluor oder Brom, eine Alkylgruppe mit 1, 2, 3 oder 4 Kohlenstoffatomen, eine Alkoxygruppe mit 1, 2 oder 3 Kohlenstoffatomen, eine Alkylthiogruppe mit 1, 2 oder 3 Kohlenstoffatomen, wobei die Gruppen vollständig oder teilweise durch identische oder verschiedene Halogenatome, wie Fluor, Chlor, Brom oder Iod, substituiert sein können; für eine Nitro-, Cyano- oder Alkoxycarbonylgruppe mit 1, 2 oder 3 Kohlenstoffatomen im Alkylrest; R₁ und R₂ je weiterhin Wassestoff bedeuten können, wobei in diesem Fall jedoch R₃ nicht Methyl bedeuten kann, sondern zusätzlich für eine Phenylgruppe steht, die 1 oder 2 Fluor-, Chlor-, Brom- oder Iodatom, Alkylgruppen mit 1, 2 , 3 oder 4 Kohlenstoffatomen, oder Alkoxygruppen mit 1, 2 oder 3 Kohlenstoffatomen tragen kann, oder für eine Phenoxygruppe, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomen tragen kann oder R₁ für Wasserstoff steht und R₂ und R₃ zusammen für eine Methylen-Dioxy-Gruppe stehen, oder zusammen mit dem Phenylring, an den sie gebunden sind, für einen Naphthalinring stehen, und wobei M für Wasserstoff, ein Alkalimetall, wie Natrium oder Kalium, oder Ammonium steht; und
(b) einen pharmazeutisch verträglichen Träger.

12. Formulierung nach Anspruch 11, dadurch gekennzeichnet, daß der Metabolit die nachfolgende Formel aufweist:

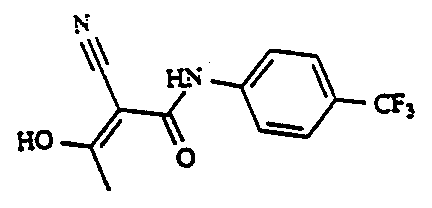

13. Pharmazeutische Formulierung nach Anspruch 11, dadurch gekennzeichnet, daß die Verbindung eine therapeutisch wirksame Menge einer Verbindung der nachfolgenden Formel aufweist: 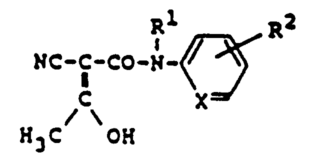 wobei

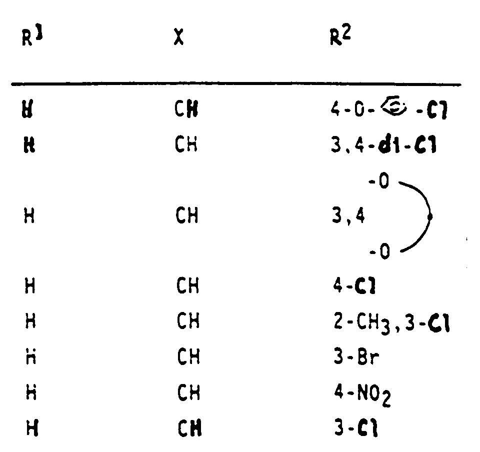

14. Verwendung
(a) einer therapeutisch wirksamen Menge einer Verbindung der nachfolgenden Formel: 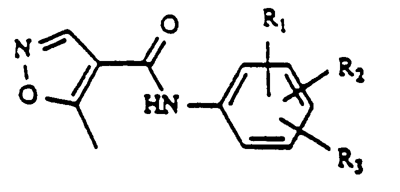 wobei R₁, R₂ und R₃, die gleich oder unterschiedlich sein können, je stehen für eine Alkylgruppe mit 1, 2 oder 3 Kohlenstoffatomen, eine Alkoxygruppe mit 1, 2 oder 3 Kohlenstoffatomen, eine Alkylthiogruppe mit 1, 2 oder 3 Kohlenstoffatomen, wobei die Gruppen teilweise oder vollständig durch identische oder unterschiedliche Halogenatome, wie Fluor-, Chlor-, Brom- oder Iodatome, substituiert sein können;
Halogenatome, wie Fluor, Chlor, Brom oder Iod;
Nitro;
Cyano;
Alkoxycarbonylgruppen mit 1, 2 oder 3 Kohlenstoffatomen im Alkylrest, und wobei R₁ und R₂ je weiterhin für Wasserstoff stehen, wobei in diesem Fall R₃ jedoch nicht Methyl bedeuten kann, sondern zusätzlich für eine Phenylgruppe stehen kann, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomen tragen kann, oder für eine Phenoxygruppe, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomem tragen kann, oder wobei R₁ für Wasserstoff steht, R₂ und R₃ zusammen für eine Methylen-Dioxy-Gruppe stehen oder sie zusammen mit dem Phenylring, an den sie gebunden sind, für einen Naphthalinring stehen; und
(b) eines pharmazeutisch verträglichen Trägers zur Herstellung einer pharmazeutischen, zur ophthalmischen Verabreichung ausgelegten Formulierung zur Behandlung von Augenerkrankungen mit Immunätiologie.

15. Verwendung einer therapeutisch wirksamen Menge einer Verbindung der nachfolgenden Formel: 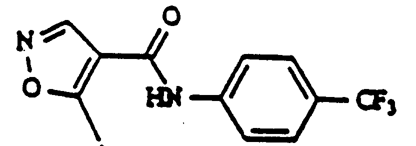 zur Herstellung einer pharmazeutischen, zur ophthalmischen Verabreichung ausgelegten Formulierung zur Behandlung von Augenerkrankungen mit Immunätiologie nach Anspruch 14.

16. Verwendung von
(a) einer therapeutisch wirksamen Menge einer Verbindung der nachfolgenden Formel: 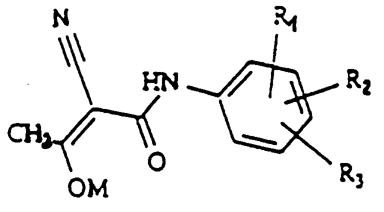 wobei R₁, R₂ und R₃, die gleich oder unterschiedlich sein können, je stehen für ein Halogen, wie Chlor, Fluor oder Brom, eine Alkylgruppe mit 1, 2, 3 oder 4 Kohlenstoffatomen, eine Alkoxygruppe mit 1, 2 oder 3 Kohlenstoffatomen, eine Alkylthiogruppe mit 1, 2 oder 3 Kohlenstoffatomen, wobei die Gruppen vollständig oder teilweise durch identische oder verschiedene Halogenatome, wie Fluor, Chlor, Brom oder Iod, substituiert sein können; für eine Nitro-, Cyano- oder Alkoxycarbonylgruppe mit 1, 2 oder 3 Kohlenstoffatomen im Alkylrest; R₁ und R₂ je weiterhin Wasserstoff bedeuten können, wobei in diesem Fall jedoch R₃ nicht Methyl bedeuten kann, sondern zusätzlich für eine Phenylgruppe steht, die 1 oder 2 Fluor-, Chlor-, Brom- oder Iodatome, Alkylgruppen mit 1, 2 oder 3 Kohlenstoffatomen, oder Alkoxygruppen mit 1, 2 oder 3 Kohlenstoffatomen tragen kann, oder für eine Phenoxygruppe, die 1 oder 2 Fluor-, Chlor-, Brom- oder Iodatome, Alkylgruppen mit 1, 2 oder 3 Kohlenstoffatomen tragen kann oder R₁ für Wasserstoff steht und R₂ und R₃ zusammen für eine Methylen-Dioxy-Gruppe stehen, oder zusammen mit dem Phenylring, an den sie gebunden sind, für einen Naphthalinring stehen, und wobei M für Wasserstoff, ein Alkalimetall, wie Natrium oder Kalium, oder Ammonium steht; und
(b) eines pharmazeutisch verträglichen Trägers zur Herstellung einer pharmazeutischen, zur ophthalmischen Verabreichung ausgelegten Formulierung zur Behandlung von Augenerkrankungen mit Immunätiologie nach Anspruch 14.

17. Verwendung einer therapeutisch wirksamen Menge einer Verbindung der nachfolgenden Formel: 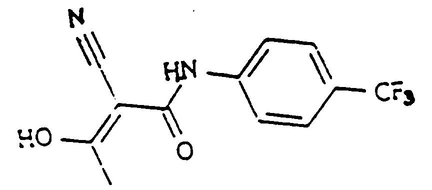 zur Herstellung einer pharmazeutischen, zur ophthalmischen Verabreichung ausgelegten Formulierung zur Behandlung von Augenerkrankungen mit Immunätionlogie nach Anspruch 16.

18. Verwendung einer therapeutisch wirksamen Menge einer Verbindung der nachfolgenden Formel: 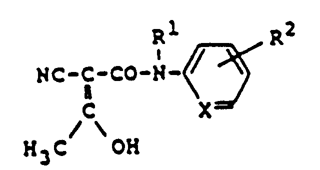 wobei 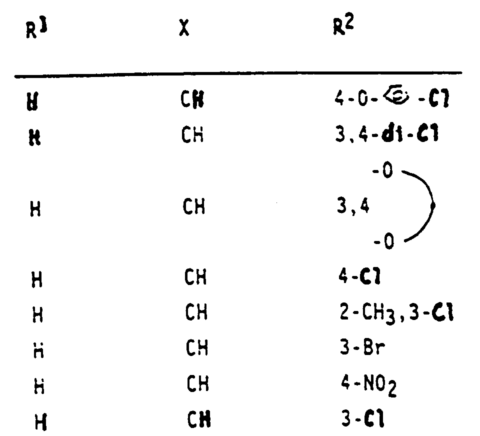 zur Herstellung einer pharmazeutischen, zur ophthalmischen Verabreichung ausgelegten Formulierung zur Behandlung von Augenerkrankungen mit Immunätiologie nach Anspruch 16.

19. Verwendung nach einem oder mehreren der Ansprüche 14-18, dadurch gekennzeichnet, daß die Verbindung in einer Konzentration von 0,0001-30 mg/kg/Tag, bevorzugt in einer Konzentration von 0,01-10,0 mg/kg/Tag, zugeführt wird.

20. Verwendung nach einem oder mehreren der Ansprüche 14-18, dadurch gekennzeichnet, daß die Verbindung dem Auge topisch in einer Konzentration von 0,05-10,0 Gew.% zugeführt wird.

21. Verwendung nach einem oder mehreren der Ansprüche 14-18, dadurch gekennzeichnet, daß die Augenerkrankung mit Immunätiologie zur Gruppe von Uveitis, Retinitis, Allergie und trockenem Auge ausgewählt wird.

22. Verwendung nach einem oder mehreren der Ansprüche 14-18, weiterhin einsetzbar zur Verlängerung der Beständigkeit von Transplantaten von Cornea- oder anderen Augengeweben und als chirurgische Zusätze bei Patienten, die atopisch sind oder an Störungen des Immunsystems leiden.

## Patentansprüche (Patentansprüche für folgende(n) Vertragsstaat(en): ES)

1. Verwendung
(a) einer therapeutisch wirksamen Menge einer Verbindung mit der Formel: 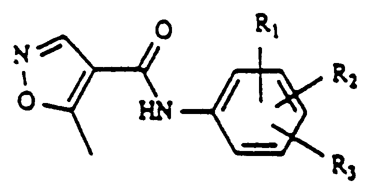 wobei R₁, R₂ und R₃, die gleich oder unterschiedlich sein können, je stehen für eine Alkylgruppe mit 1, 2 oder 3 Kohlenstoffatomen, eine Alkoxygruppe mit 1, 2 oder 3 Kohlenstoffatomen, eine Alkylthiogruppe mit 1, 2 oder 3 Kohlenstoffatomen, wobei die Gruppen teilweise oder vollständig durch identische oder unterschiedliche Halogenatome, wie Fluor-, Chlor-, Brom- oder Iodatome, substituiert sein können;
Halogenatome, wie Fluor, Chlor, Brom oder Iod;
Nitro;
Cyano;
Alkoxycarbonylgruppen mit 1, 2 oder 3 Kohlenstoffatomen im Alkylrest, und wobei R₁ und R₂ je weiterhin für Wasserstoff stehen, wobei in diesem Fall R₃ jedoch nicht Methyl bedeuten kann, sondern zusätzlich für eine Phenylgruppe stehen kann, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomen tragen kann, oder für eine Phenoxygruppe, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomen tragen kann, oder wobei R₁ für Wasserstoff steht, R₂ und R₃ zusammen für eine Methylen-Dioxy-Gruppe stehen oder sie zusammen mit dem Phenylring, an den sie gebunden sind, für einen Naphthalinring stehen; und
(b) eines pharmazeutisch verträglichen Trägers zur Herstellung einer pharmazeutischen Formulierung zur Behandlung von Augenerkrankungen mit Immunätiologie.

2. Verwendung einer therapeutisch wirksamen Menge einer Verbindung der Formel: 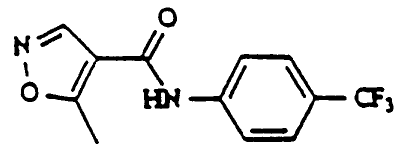 zur Herstellung einer pharmazeutischen Zubereitung zur Behandlung von Augenerkrankungen mit Immunätiologie nach Anspruch 1.

3. Verwendung
(a) einer therapeutisch wirksamen Menge einer Verbindung der Formel: 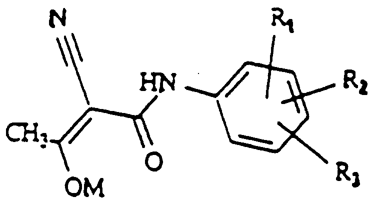 wobei R₁, R₂ und R₃, die gleich oder unterschiedlich sein können, je stehen für ein Halogen, wie Chlor, Fluor oder Brom, eine Alkylgruppe mit 1, 2, 3 oder 4 Kohlenstoffatomen, eine Alkoxygruppe mit 1, 2 oder 3 Kohlenstoffatomen, eine Alkylthiogruppe mit 1, 2 oder 3 Kohlenstoffatomen, wobei die Gruppen vollständig oder teilweise durch identische oder verschiedene Halogenatome, wie Fluor, Chlor, Brom oder Iod, substituiert sein können; für eine Nitro-, Cyano- oder Alkoxycarbonylgruppe mit 1, 2 oder 3 Kohlenstoffatomen im Alkylrest; R₁ und R₂ je weiterhin Wasserstoff bedeuten können, wobei in diesem Fall jedoch R₃ nicht Methyl bedeuten kann, sondern zusätzlich für eine Phenylgruppe steht, die 1 oder 2 Fluor-, Chlor-, Brom- oder Iodatome, Alkylgruppen mit 1, 2, 3 oder 4 Kohlenstoffatomen, oder Alkoxygruppen mit 1, 2 oder 3 Kohlenstoffatomen tragen kann, oder für eine Phenoxygruppe, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomen tragen kann oder R₁ für Wasserstoff steht und R₂ und R₃ zusammen für eine Methylen-Dioxy-Gruppe stehen, oder zusammen mit dem Phenylring, an den sie gebunden sind, für einen Naphthalinring stehen, und wobei M für Wasserstoff, ein Alkalimetall, wie Natrium oder Kalium, oder Ammonium steht; und
(b) eines pharmazeutisch verträglichen Trägers zur Herstellung einer pharmazeutischen Formulierung zur Behandlung von Augenerkrankungen mit Immunätiologie nach Anspruch 1.

4. Verwendung einer therapeutisch wirksamen Menge einer Verbindung der Formel: 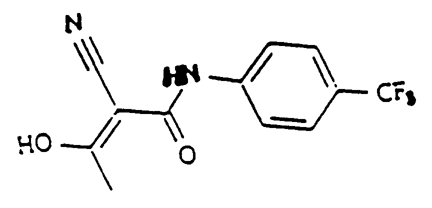 zur Herstellung einer pharmazeutischen Formulierung zur Behandlung von Augenerkrankungen mit Immunätiologie nach Anspruch 3.

5. Verwendung einer therapeutisch wirksamen Menge einer Verbindung der Formel: 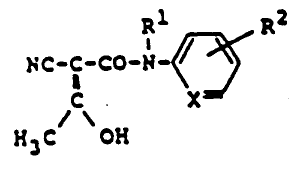 wobei 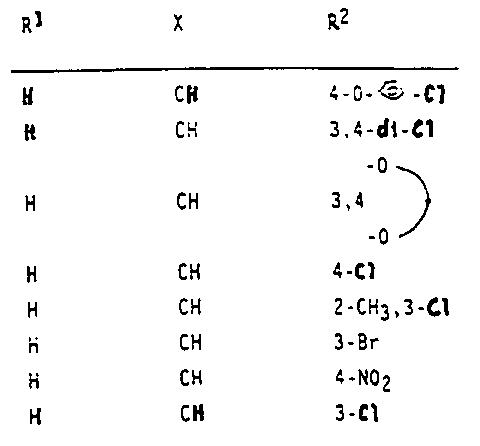 zur Herstellung einer pharmazeutischen Formulierung zur Behandlung von Augenerkrankungen mit Immunätiologie nach Anspruch 3.

6. Verwendung nach einem oder mehreren der Ansprüche 1-5, dadurch gekennzeichnet, daß die Verbindung in einer Konzentration von 0,0001-30 mg/kg/Tag, bevorzugt in einer Konzentration von 0,01-10,0 mg/kg/Tag, zugeführt wird.

7. Verwendung nach einem oder mehreren der Ansprüche 1-6, dadurch gekennzeichnet, daß die Augenerkrankung mit Immunätiologie zur Gruppe von Uveitis, Retinitis, Allergie und trockenem Auge ausgewählt wird.

8. Verwendung nach einem oder mehreren der Ansprüche 1-6, weiterhin einsetzbar zur Verlängerung der Beständigkeit von Transplantaten von Cornea- oder anderen Augengeweben und als chirurgische Zusätze bei Patienten, die atopisch sind oder an Störungen des Immunsystems leiden.

9. Verfahren zur Herstellung einer pharmazeutischen Formulierung, umfassend das Vermischen:
(a) einer therapeutische wirksamen Menge einer Verbindung mit der Formel: 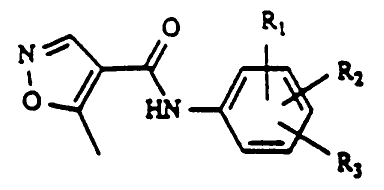 wobei R₁, R₂ und R₃, die gleich oder unterschiedlich sein können, je stehen für eine Alkylgruppe mit 1, 2 oder 3 Kohlenstoffatomen, eine Alkoxygruppe mit 1, 2 oder 3 Kohlenstoffatomen, eine Alkylthiogruppe mit 1, 2 oder 3 Kohlenstoffatomen, wobei die Gruppen teilweise oder vollständig durch identische oder unterschiedliche Halogenatome, wie Fluor-, Chlor-, Brom- oder Iodatome, substituiert sein können;
Halogenatome, wie Fluor, Chlor, Brom oder Iod;
Nitro;
Cyano;
Alkoxycarbonylgruppen mit 1, 2 oder 3 Kohlenstoffatomen im Alkylrest, und wobei R₁ und R₂ je weiterhin für Wasserstoff stehen, wobei in diesem Fall R₃ jedoch nicht Methyl bedeuten kann, sondern zusätzlich für eine Phenylgruppe stehen kann, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomen tragen kann, oder für eine Phanoxygruppe, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomen tragen kann, oder wobei R₁ für Wasserstoff steht, R₂ und R₃ zusammen für eine Methylen-Dioxy-Gruppe stehen oder sie zusammen mit dem Phenylring, an den sie gebunden sind, für einen Naphthalinring stehen; und
(b) eines pharmazeutisch verträglichen Trägers, um eine pharmazeutische, zur ophthalmischen Verabreichung ausgelegte Forumlierung zu erhalten.

10. Verfahren nach Anspruch 9, dadurch gekennzeichnet, daß die Verbindung eine therapeutisch wirksame Menge einer Verbindung der nachfolgenden Formel darstellt:

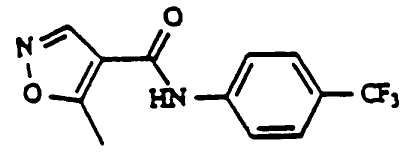

11. Verfahren nach Anspruch 9, dadurch gekennzeichnet, daß es umfaßt das Vermischen von:
(a) einer therapeutisch wirksamen Menge eines Metaboliten einer Verbindung des Anspruchs 9, wobei der Metabolit nachfolgende Formel aufweist: 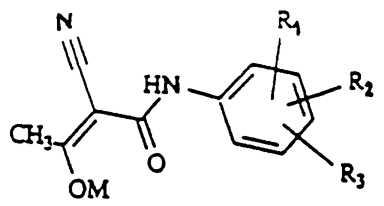 wobei R₁, R₂ und R₃, die gleich oder unterschiedlich sein können, je stehen für ein Halogen, wie Chlor, Fluor oder Brom, eine Alkylgruppe mit 1, 2, 3 oder 4 Kohlenstoffatomen, eine Alkoxygruppe mit 1, 2 oder 3 Kohlenstoffatomen, eine Alkylthiogruppe mit 1, 2 oder 3 Kohlenstoffatomen, wobei die Gruppen vollständig oder teilweise durch identische oder verschiedene Halogenatome, wie Fluor, Chlor, Brom oder Iod, substituiert sein können; für eine Nitro-, Cyano- oder Alkoxycarbonylgruppe mit 1, 2 oder 3 Kohlenstoffatomen im Alkylrest; R₁ und R₂ je weiterhin Wassestoff bedeuten können, wobei in diesem Fall jedoch R₃ nicht Methyl bedeuten kann, sondern zusätzlich für eine Phenylgruppe steht, die 1 oder 2 Fluor-, Chlor-, Brom- oder Iodatom, Alkylgruppen mit 1, 2 , 3 oder 4 Kohlenstoffatomen, oder Alkoxygruppen mit 1, 2 oder 3 Kohlenstoffatomen tragen kann, oder für eine Phenoxygruppe, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomen tragen kann oder R₁ für Wasserstoff steht und R₂ und R₃ zusammen für eine Methylen-Dioxy-Gruppe stehen, oder zusammen mit dem Phenylring, an den sie gebunden sind, für einen Naphthalinring stehen, und wobei M für Wasserstoff, ein Alkalimetall, wie Natrium oder Kalium, oder Ammonium steht; und
(b) eines pharmazeutisch verträglichen Trägers, um eine pharmazeutische, zur ophthalmischen Verbabreichung ausgelegte Formulierung zu erhalten.

12. Verfahren nach Anspruch 11, dadurch gekennzeichnet, daß der Metabolit die nachfolgende Formel aufweist:

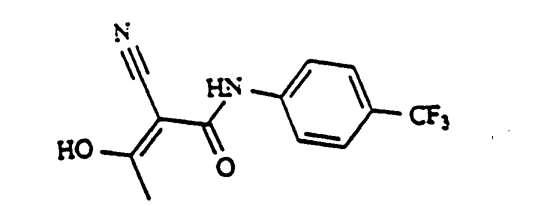

13. Verfahren nach Anspruch 11, dadurch gekennzeichnet, daß die Verbindung eine therapeutisch wirksame Menge einer Verbindung der nachfolgenden Formel aufweist: 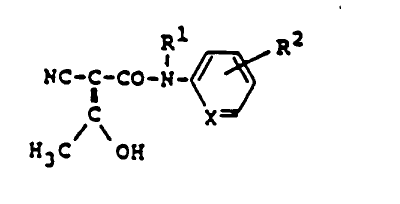 wobei

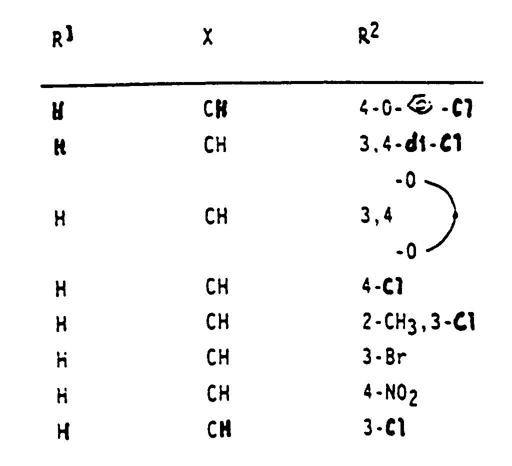

14. Verwendung
(a) einer therapeutisch wirksamen Menge einer Verbindung der nachfolgenden Formel: 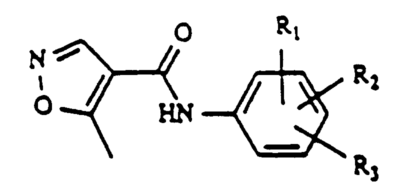 wobei R₁, R₂ und R₃, die gleich oder unterschiedlich sein können, je stehen für eine Alkylgruppe mit 1, 2 oder 3 Kohlenstoffatomen, eine Alkoxygruppe mit 1, 2 oder 3 Kohlenstoffatomen, eine Alkylthiogruppe mit 1, 2 oder 3 Kohlenstoffatomen, wobei die Gruppen teilweise oder vollständig durch identische oder unterschiedliche Halogenatome, wie Fluor-, Chlor-, Brom- oder Iodatome, substituiert sein können;
Halogenatome, wie Fluor, Chlor, Brom oder Iod;
Nitro;
Cyano;
Alkoxycarbonylgruppen mit 1, 2 oder 3 Kohlenstoffatomen im Alkylrest, und wobei R₁ und R₂ je weiterhin für Wasserstoff stehen, wobei in diesem Fall R₃ jedoch nicht Methyl bedeuten kann, sondern zusätzlich für eine Phenylgruppe stehen kann, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomen tragen kann, oder für eine Phenoxygruppe, die 1 oder 2 Fluor-, Chlor-, Brom- oder Iodatome, Alkylgruppen mit 1, 2 oder 3 Kohlenstoffatomen oder Alkoxygruppen mit 1, 2 oder 3 Kohlenstoffatomem tragen kann, oder wobei R₁ für Wasserstoff steht, R₂ und R₃ zusammen für eine Methylen-Dioxy-Gruppe stehen oder sie zusammen mit dem Phenylring, an den sie gebunden sind, für einen Naphthalinring stehen; und
(b) eines pharmazeutisch verträglichen Trägers zur Herstellung einer pharmazeutischen, zur ophthalmischen Verabreichung ausgelegten Formulierung zur Behandlung von Augenerkrankungen mit Immunätiologie.

15. Verwendung einer therapeutisch wirksamen Menge einer Verbindung der nachfolgenden Formel: 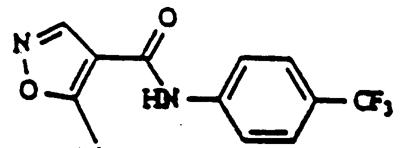 zur Herstellung einer pharmazeutischen, zur ophthalmischen Verabreichung ausgelegten Formulierung zur Behandlung von Augenerkrankungen mit Immunätiologie nach Anspruch 14.

16. Verwendung von
(a) einer therapeutisch wirksamen Menge einer Verbindung der nachfolgenden Formel: 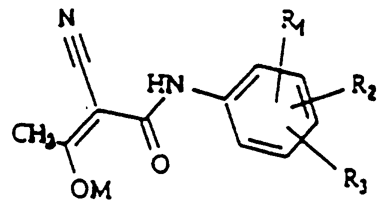 wobei R₁, R₂ und R₃, die gleich oder unterschiedlich sein können, je stehen für ein Halogen, wie Chlor, Fluor oder Brom, eine Alkylgruppe mit 1, 2, 3 oder 4 Kohlenstoffatomen, eine Alkoxygruppe mit 1, 2 oder 3 Kohlenstoffatomen, eine Alkylthiogruppe mit 1, 2 oder 3 Kohlenstoffatomen, wobei die Gruppen vollständig oder teilweise durch identische oder verschiedene Halogenatome, wie Fluor, Chlor, Brom oder Iod, substituiert sein können; für eine Nitro-, Cyano- oder Alkoxycarbonylgruppe mit 1, 2 oder 3 Kohlenstoffatomen im Alkylrest; R₁ und R₂ je weiterhin Wasserstoff bedeuten können, wobei in diesem Fall jedoch R₃ nicht Methyl bedeuten kann, sondern zusätzlich für eine Phenylgruppe steht, die 1 oder 2 Fluor-, Chlor-, Brom- oder Iodatome, Alkylgruppen mit 1, 2 oder 3 Kohlenstoffatomen, oder Alkoxygruppen mit 1, 2 oder 3 Kohlenstoffatomen tragen kann, oder für eine Phenoxygruppe, die 1 oder 2 Fluor-, Chlor-, Brom- oder Iodatome, Alkylgruppen mit 1, 2 oder 3 Kohlenstoffatomen tragen kann oder R₁ für Wasserstoff steht und R₂ und R₃ zusammen für eine Methylen-Dioxy-Gruppe stehen, oder zusammen mit dem Phenylring, an den sie gebunden sind, für einen Naphthalinring stehen, und wobei M für Wasserstoff, ein Alkalimetall, wie Natrium oder Kalium, oder Ammonium steht; und
(b) eines pharmazeutisch verträglichen Trägers zur Herstellung einer pharmazeutischen, zur ophthalmischen Verabreichung ausgelegten Formulierung zur Behandlung von Augenerkrankungen mit Immunätiologie nach Anspruch 14.

17. Verwendung einer therapeutisch wirksamen Menge einer Verbindung der nachfolgenden Formel: 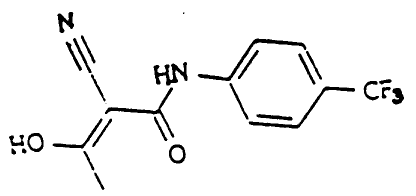 zur Herstellung einer pharmazeutischen, zur ophthalmischen Verabreichung ausgelegten Formulierung zur Behandlung von Augenerkrankungen mit Immunätionlogie nach Anspruch 16.

18. Verwendung einer therapeutisch wirksamen Menge einer Verbindung der nachfolgenden Formel: 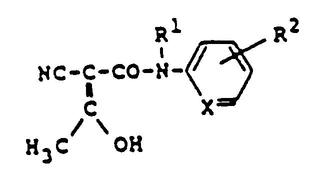 wobei 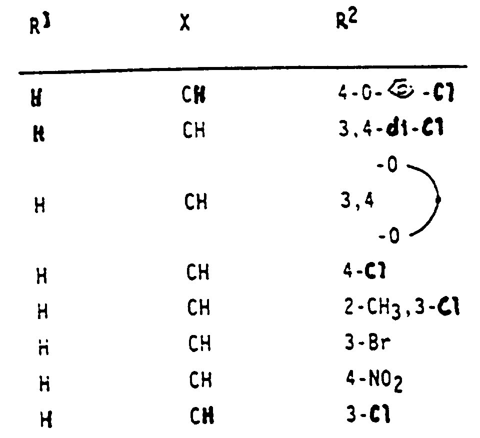 zur Herstellung einer pharmazeutischen, zur ophthalmischen Verabreichung ausgelegten Formulierung zur Behandlung von Augenerkrankungen mit Immunätiologie nach Anspruch 16.

19. Verwendung nach einem oder mehreren der Ansprüche 14-18, dadurch gekennzeichnet, daß die Verbindung in einer Konzentration von 0,0001-30 mg/kg/Tag, bevorzugt in einer Konzentration von 0,01-10,0 mg/kg/Tag, zugeführt wird.

20. Verwendung nach einem oder mehreren der Ansprüche 14-18, dadurch gekennzeichnet, daß die Verbindung dem Auge topisch in einer Konzentration von 0,05-10,0 Gew.% zugeführt wird.

21. Verwendung nach einem oder mehreren der Ansprüche 14-18, dadurch gekennzeichnet, daß die Augenerkrankung mit Immunätiologie zur Gruppe von Uveitis, Retinitis, Allergie und trockenem Auge ausgewählt wird.

22. Verwendung nach einem oder mehreren der Ansprüche 14-18, weiterhin einsetzbar zur Verlängerung der Beständigkeit von Transplantaten von Cornea- oder anderen Augengeweben und als chirurgische Zusätze bei Patienten, die atopisch sind oder an Störungen des Immunsystems leiden.

## Revendications (Revendications pour l'(les) Etat(s) contractant(s) suivant(s): AT, BE, DE, DK, FR, GB, IT, LI, LU, NL, SE)

1. Utilisation d'
a) une quantité efficace thérapeutiquement d'un composé ayant la formule : 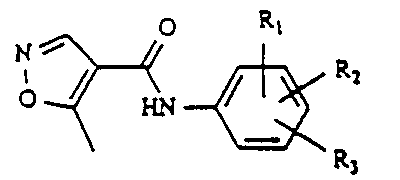 dans laquelle : R₁, R₂ et R₃, qui peuvent être identiques ou différents, représentent chacun un groupe alkyle ayant 1, 2 ou 3 atomes de carbone, un groupe alkoxy ayant 1, 2 ou 3 atomes de carbone, un groupe alkylthio de 1, 2 ou 3 atomes de carbone, ces groupes pouvant être substitués en partie ou entièrement par des atomes d'halogène identiques ou différents, tels que des atomes de fluor, de chlore, de brome ou d'iode ;
des atomes d'halogène, tels que le fluor, le chlore, le brome ou l'iode ;
un groupe nitro ;
un groupe cyano ;
des groupes alkoxycarbonyle ayant 1, 2 ou 3 atomes de carbone dans la partie alkyle, et dans laquelle R₁ et R₂ représentent chacun en outre l'hydrogène, cas dans lequel, cependant, R₃ ne peut pas représenter le méthyle, mais peut représenter en outre un groupe phényle qui peut porter un ou deux atomes de fluor, de chlore, de brome ou d'iode, des groupes alkyle ayant 1, 2 ou 3 atomes de carbone ou des groupes alkoxy ayant 1, 2 ou 3 atomes de carbone, ou peut représenter un groupe phénoxy qui peut porter un ou deux atomes de fluor, de chlore, de brome ou d'iode, des groupes alkyle ayant 1, 2 ou 3 atomes de carbone ou des groupes alkyoxy ayant 1, 2 ou 3 atomes de carbone, ou dans laquelle R₁ représente l'hydrogène, R₂ et R₃ représentent ensemble un groupe méthylène-dioxy ou, conjointement avec l'anneau phényle auxquels ils sont liés, ils représentent un anneau de naphtalène ; et
b) un porteur acceptable pharmaceutiquement pour la préparation d'une formulation pharmaceutiquement permettant de traiter des maladies oculaires associées à une étiologie immune.

2. Utilisation d'une quantité efficace thérapeutiquement d'un composé de la formule : 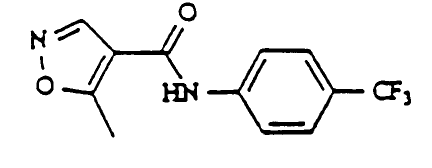 pour la préparation d'une formulation pharmaceutique permettant de traiter des maladies oculaires associées à une étiologie immune selon la revendication 1.

3. Utilisation de
a) une quantité efficace thérapeutiquement d'un composé ayant la formule : 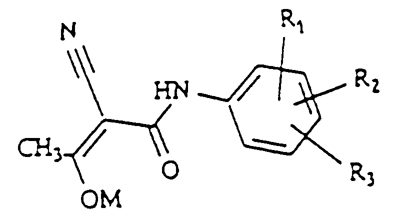 dans laquelle R₁, R₂ et R₃, qui peuvent être identiques ou différents, représentent chacun un halogène, tel que le chlore, le fluor, ou le brome, un groupe alkyle ayant 1, 2, 3 ou 4 atomes de carbone, un groupe alkoxy ayant 1, 2 ou 3 atomes de carbone, un groupe alkylthio de 1, 2 ou 3 atomes de carbone, ces groupes pouvant être substitués entièrement ou en partie par des atomes d'halogène identiques ou différents, tels que des atomes de fluor, de chlore, de brome ou d'iode ; un groupe nitro, cyano ou alkoxycarbonyle ayant 1, 2 ou 3 atomes de carbone dans la partie alkyle ; R₁ et R₂ représentent chacun en outre l'hydrogène, cas dans lequel, cependant, R₃ ne peut pas représenter le méthyle, mais peut représenter en outre un groupe phényle qui peut porter un ou deux atomes de fluor, de chlore, de brome ou d'iode, des groupes alkyle ayant 1, 2, 3 ou 4 atomes de carbone ou des groupes alkoxy ayant 1, 2 ou 3 atomes de carbone, ou peut représenter un groupe phénoxy qui peut porter un ou deux atomes de fluor, de chlore, de brome ou d'iode, des groupes alkyle ayant 1, 2 ou 3 atomes de carbone ou des groupes alkyoxy ayant 1, 2 ou 3 atomes de carbone, ou R₁ représente l'hydrogène, et R₂ et R₃ représentent ensemble un groupe méthylène-dioxy ou, conjointement avec l'anneau phényle auxquels ils sont liés, ils représentent un anneau de naphtalène, et dans laquelle M représente l'hydrogène, un métal alcalin, tel que le sodium ou le potassium, ou l'ammonium ; et
b) un porteur acceptable pharmaceutiquement pour la préparation d'une formulation pharmaceutique permettant de traiter des maladies oculaires associées à une étiologie immune selon la revendication 1.

4. Utilisation d'une quantité efficace thérapeutiquement d'un composée ayant la formule : 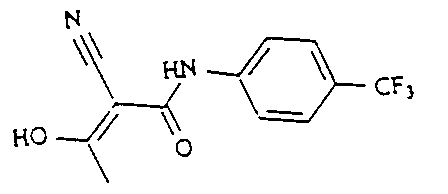 pour la préparation d'une formulation pharmaceutique permettant de traiter des maladies oculaires associées à une étiologie immune selon la revendication 3.

5. Utilisation d'une quantité efficace thérapeutiquement d'un composé ayant la formule : 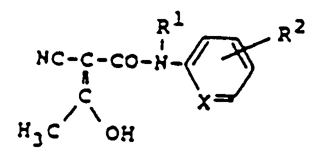 dans laquelle : 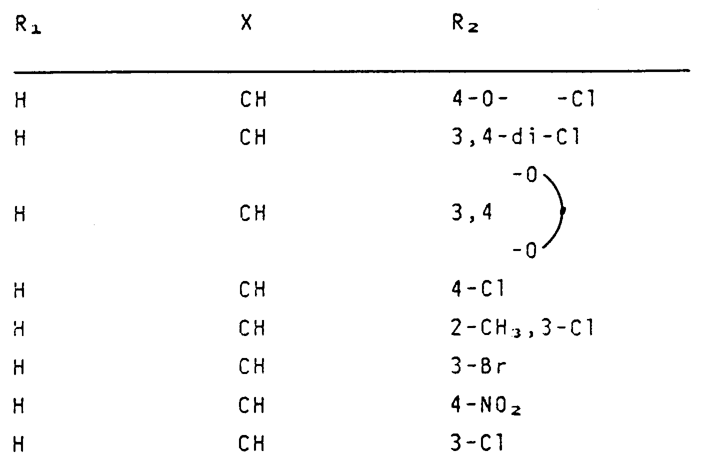 pour la préparation d'une formulation pharmaceutique permettant de traiter des maladies oculaires associées à une étiologie immune selon la revendication 3.

6. Utilisation selon une ou plusieurs des revendications 1 à 5, caractérisée en ce que le composé est fourni à une concentration comprise dans la plage allant de 0,0001 à 30 mg/kg/jour, de préférence à une concentration comprise dans la plage allant de 0,01 à 10,0 mg/kg/jour.

7. Utilisation selon une ou plusieurs des revendications 1 à 6, caractérisée en ce que la maladie oculaire associée à une étiologie immune est sélectionnée dans le groupe comprenant l'uvéite, la rétinite, l'allergie et l'oeil sec.

8. Utilisation selon une ou plusieurs des revendications 1 à 6, servant à prolonger la durée de vie d'une greffe de la cornée ou d'un autre tissu oculaire et servant d'ajouts chirurgicaux chez des patients qui sont atopiques ou souffrent d'une immunité déficiente.

9. Formulation pharmaceutique adaptée pour une administration ophtalmique, caractérisée par le fait de comprendre :
a) une quantité efficace thérapeutiquement d'un composé ayant la formule : 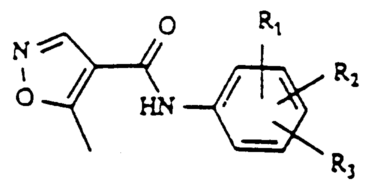 dans laquelle : R₁, R₂ et R₃, qui peuvent être identiques ou différents, représentent chacun un groupe alkyle ayant 1, 2 ou 3 atomes de carbone, un groupe alkoxy ayant 1, 2 ou 3 atomes de carbone, un groupe alkylthio de 1, 2 ou 3 atomes de carbone, ces groupes pouvant être substitués en partie ou entièrement par des atomes d'halogène identiques ou différents, tels que des atomes de fluor, de chlore, de brome ou d'iode ;
des atomes d'halogène, tels que le fluor, le chlore, le brome ou l'iode ;
un groupe nitro ;
un groupe cyano ;
des groupes alkoxycarbonyle ayant 1, 2 ou 3 atomes de carbone dans la partie alkyle, et dans laquelle R₁ et R₂ représentent chacun en outre l'hydrogène, cas dans lequel, cependant, R₃ ne peut pas représenter le méthyle, mais peut représenter en outre un groupe phényle qui peut porter un ou deux atomes de fluor, de chlore, de brome ou d'iode, des groupes alkyle ayant 1, 2 au 3 atomes de carbone ou des groupes alkoxy ayant 1, 2 ou 3 atomes de carbone, ou peut représenter un groupe phénoxy qui peut porter un ou deux atomes de fluor, de chlore, de brome ou d'iode, des groupes alkyle ayant 1, 2 ou 3 atomes de carbone ou des groupes alkyoxy ayant 1, 2 ou 3 atomes de carbone, ou dans laquelle R₁ représente l'hydrogène, R₂ et R₃ représentent ensemble un groupe méthylène-dioxy ou, conjointement avec l'anneau phényle auxquels ils sont liés, ils représentent un anneau de naphtalène ; et
b) un porteur acceptable pharmaceutiquement.

10. Formulation selon la revendication 9, caractérisée en ce que le composé est une quantité efficace thérapeutiquement d'un composé ayant la formule :

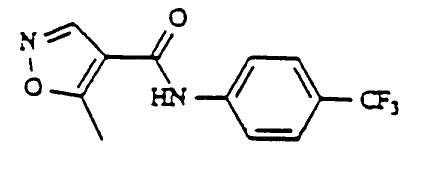

11. Formulation pharmaceutique selon la revendication 9, caractérisée par le fait de comprendre :
a) une quantité efficace thérapeutiquement d'un métabolite d'un composé selon la revendication 9, ledit métabolite ayant la formule : 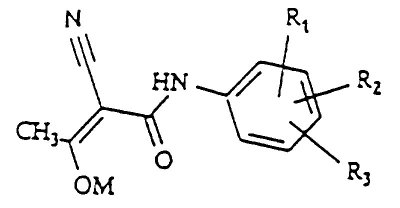 dans laquelle R₁, R₂ et R₃, qui peuvent être identiques ou différents, représentent chacun un halogène, tel que le chlore, le fluor, ou le brome, un groupe alkyle ayant 1, 2, 3 ou 4 atomes de carbone, un groupe alkoxy ayant 1, 2 ou 3 atomes de carbone, un groupe alkylthio de 1, 2 ou 3 atomes de carbone, ces groupes pouvant être substitués entièrement ou en partie par des atomes d'halogène identiques ou différents, tels que le fluor, le chlore, le brome ou l'iode ; un groupe nitro, cyano ou alkoxycarbonyle ayant 1, 2 ou 3 atomes de carbone dans la partie alkyle ; R₁ et R₂ représentent chacun en outre l'hydrogène, cas dans lequel, cependant, R₃ ne peut pas représenter le méthyle, mais peut représenter en outre un groupe phényle qui peut porter un ou deux atomes de fluor, de chlore, de brome ou d'iode, des groupes alkyle ayant 1, 2, 3 au 4 atomes de carbone ou des groupes alkoxy ayant 1, 2 ou 3 atomes de carbone, ou peut représenter un groupe phénoxy qui peut porter un ou deux atomes de fluor, de chlore, de brome ou d'iode, des groupes alkyle ayant 1, 2 ou 3 atomes de carbone ou des groupes alkyoxy ayant 1, 2 ou 3 atomes de carbone, ou R₁ représente l'hydrogène, et R₂ et R₃ représentent ensemble un groupe méthylène-dioxy ou, conjointement avec l'anneau phényle auxquels ils sont liés, ils représentent un anneau de naphtalène, et dans laquelle M représente l'hydrogène, un métal alcalin, tel que le sodium ou le potassium, ou l'ammonium ; et
b) un porteur acceptable pharmaceutiquement.

12. Formulation selon la revendication 11, caractérisée en ce que le métabolite présente la formule :

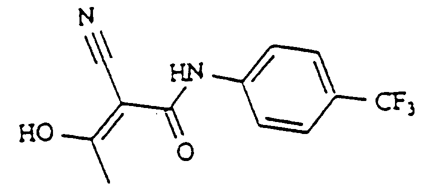

13. Formulation pharmaceutique selon la revendication 11, caractérisée en ce que le composé est une quantité efficace thérapeutiquement d'un composé ayant la formule : 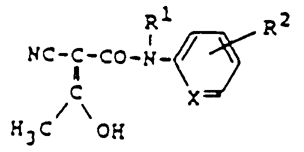 dans laquelle :

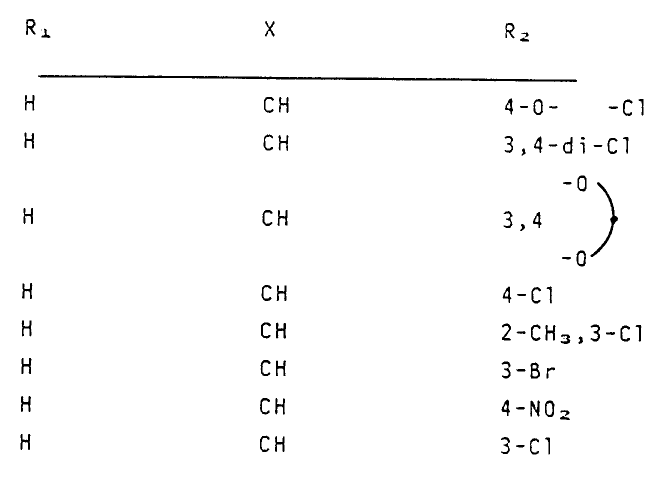

14. Utilisation d'
a) une quantité efficace thérapeutiquement d'un composé ayant la formule : 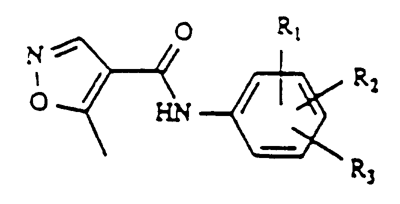 dans laquelle : R₁, R₂ et R₃, qui peuvent être identiques ou différents, représentent chacun un groupe alkyle ayant 1, 2 ou 3 atomes de carbone, un groupe alkoxy ayant 1, 2 ou 3 atomes de carbone, un groupe alkylthio de 1, 2 ou 3 atomes de carbone, ces groupes pouvant être substitués en partie ou entièrement par des atomes d'halogène identiques ou différents, tels que des atomes de fluor, de chlore, de brome ou d'iode ;
des atomes d'halogène, tels que le fluor, le chlore, le brome ou l'iode ;
un groupe nitro ;
un groupe cyano ;
des groupes alkoxycarbonyle ayant 1, 2 ou 3 atomes de carbone dans la partie alkyle, et dans laquelle R₁ et R₂ représentent chacun en outre l'hydrogène, cas dans lequel, cependant, R₃ ne peut pas représenter le méthyle, mais peut représenter en outre un groupe phényle qui peut porter un ou deux atomes de fluor, de chlore, de brome ou d'iode, des groupes alkyle ayant 1, 2 ou 3 atomes de carbone ou des groupes alkoxy ayant 1, 2 ou 3 atomes de carbone, ou peut représenter un groupe phénoxy qui peut porter un ou deux atomes de fluor, de chlore, de brome ou d'iode, des groupes alkyle ayant 1, 2 au 3 atomes de carbone ou des groupes alkyoxy ayant 1, 2 ou 3 atomes de carbone, ou dans laquelle R₁ représente l'hydrogène, R₂ et R₃ représentent ensemble un groupe méthylène-dioxy ou, conjointement avec l'anneau phényle auxquels ils sont liés, ils représentent un anneau de naphtalène ; et
b) un porteur acceptable pharmaceutiquement pour la préparation d'une formulation pharmaceutique, adaptée pour une administration ophtalmique, permettant de traiter des maladies oculaires associées à une étiologie immune.

15. Utilisation d'une quantité efficace thérapeutiquement d'un composé de la formule : 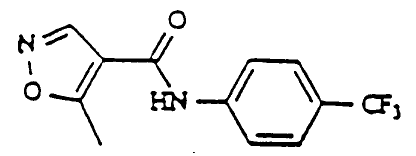 pour la préparation d'une formulation pharmaceutique, adaptée pour une administration ophtalmique, permettant de traiter des maladies oculaires associées à une étiologie immune selon la revendication 14.

16. Utilisation d'
a) une quantité efficace thérapeutiquement d'un composé ayant la formule : 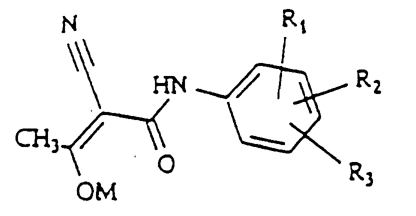 dans laquelle R₁, R₂ et R₃, qui peuvent être identiques ou différents, représentent chacun un halogène, tel que le chlore, le fluor, ou le brome, un groupe alkyle ayant 1, 2, 3 ou 4 atomes de carbone, un groupe alkoxy ayant 1, 2 ou 3 atomes de carbone, un groupe alkylthio de 1, 2 au 3 atomes de carbone, ces groupes pouvant être substitués entièrement ou en partie par des atomes d'halogène identiques ou différents, tels que le fluor, le chlore, le brome ou l'iode ; un groupe nitro, cyano ou alkoxycarbonyle ayant 1, 2 ou 3 atomes de carbone dans la partie alkyle ; R₁ et R₂ représentent chacun en outre l'hydrogène, cas dans lequel, cependant, R₃ ne peut pas représenter le méthyle, mais peut représenter en outre un groupe phényle qui peut porter un ou deux atomes de fluor, de chlore, de brome ou d'iode, des groupes alkyle ayant 1, 2, 3 ou 4 atomes de carbone ou des groupes alkoxy ayant 1, 2 ou 3 atomes de carbone, ou peut représenter un groupe phénoxy qui peut porter un ou deux atomes de fluor, de chlore, de brome ou d'iode, des groupes alkyle ayant 1, 2 ou 3 atomes de carbone ou des groupes alkyoxy ayant 1, 2 ou 3 atomes de carbone, ou R₁ représente l'hydrogène, et R₂ et R₃ représentent ensemble un groupe méthylène-dioxy ou, conjointement avec l'anneau phényle auxquels ils sont liés, ils représentent un anneau de naphtalène, et dans laquelle M représente l'hydrogène, un métal alcalin, tel que le sodium ou le potassium, ou l'ammonium ; et
b) un porteur acceptable pharmaceutiquement pour la préparation d'une formulation pharmaceutique, adaptée pour une administration ophtalmique, permettant de traiter des maladies oculaires associées à une étiologie immune selon la revendication 14.

17. Utilisation d'une quantité efficace thérapeutiquement d'un composé ayant la formule : 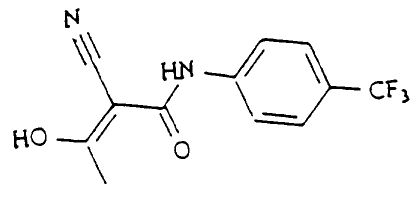 pour la préparation d'une formulation pharmaceutique, adaptée pour une administration ophtalmique, permettant de traiter des maladies oculaires associées à une étiologie immune selon la revendication 16.

18. Utilisation d'une quantité efficace thérapeutiquement d'un composé ayant la formule : 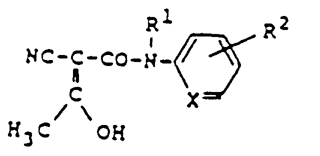 dans laquelle : 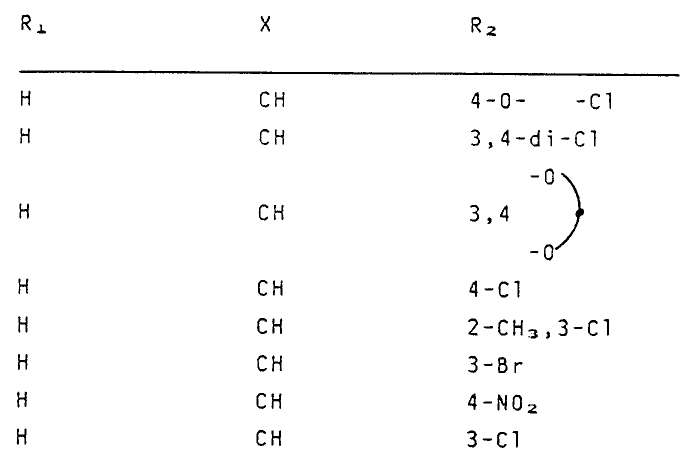 pour la préparation d'une formulation pharmaceutique, adaptée pour une administration ophtalmique, permettant de traiter des maladies oculaires associées à une étiologie immune selon la revendication 16.

19. Utilisation selon une ou plusieurs des revendications 14 à 18, caractérisée en ce que le composé est fourni à une concentration comprise dans la plage allant de 0,0001 à 30 mg/kg/jour, de préférence à une concentration comprise dans la plage allant de 0,01 à 10,0 mg/kg/jour.

20. Utilisation selon une ou plusieurs des revendications 14 à 18, caractérisée en ce que le composé est fourni localement dans l'oeil à une concentration comprise dans la plage allant de 0,05 à 10,0 % en poids.

21. Utilisation selon une ou plusieurs des revendications 14 à 18, caractérisée en ce que la maladie oculaire associée à une étiologie immune est sélectionnée dans le groupe comprenant l'uvéite, la rétinite, l'allergie et l'oeil sec.

22. Utilisation selon une ou plusieurs des revendications 14 à 18, servant en outre à prolonger la durée de vie d'une greffe de la cornée ou d'un autre tissu oculaire et servant d'ajouts chirurgicaux chez des patients qui sont atopiques ou souffrent d'une immunité déficiente.

## Revendications (Revendications pour l'(les) Etat(s) contractant(s) suivant(s): ES)

1. Utilisation d'
a) une quantité efficace thérapeutiquement d'un composé ayant la formule : 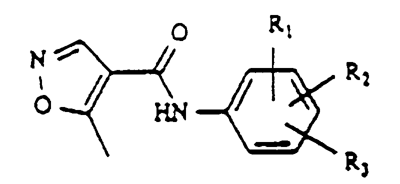 dans laquelle : R₁, R₂ et R₃, qui peuvent être identiques ou différents, représentent chacun un groupe alkyle ayant 1, 2 ou 3 atomes de carbone, un groupe alkoxy ayant 1, 2 ou 3 atomes de carbone, un groupe alkylthio de 1, 2 ou 3 atomes de carbone, ces groupes pouvant être substitués en partie ou entièrement par des atomes d'halogène identiques ou différents, tels que des atomes de fluor, de chlore, de brome ou d'iode ;
des atomes d'halogène, tels que le fluor, le chlore, le brome ou l'iode ;
un groupe nitro ;
un groupe cyano ;
des groupes alkoxycarbonyle ayant 1, 2 ou 3 atomes de carbone dans la partie alkyle, et dans laquelle R₁ et R₂ représentent chacun en outre l'hydrogène, cas dans lequel, cependant, R₃ ne peut pas représenter le méthyle, mais peut représenter en outre un groupe phényle qui peut porter un ou deux atomes de fluor, de chlore, de brome ou d'iode, des groupes alkyle ayant 1, 2 ou 3 atomes de carbone ou des groupes alkoxy ayant 1, 2 ou 3 atomes de carbone, ou peut représenter un groupe phénoxy qui peut porter un ou deux atomes de fluor, de chlore, de brome ou d'iode, des groupes alkyle ayant 1, 2 ou 3 atomes de carbone ou des groupes alkyoxy ayant 1, 2 ou 3 atomes de carbone, ou dans laquelle R₁ représente l'hydrogène, R₂ et R₃ représentent ensemble un groupe méthylène-dioxy ou, conjointement avec l'anneau phényle auxquels ils sont liés, ils représentent un anneau de naphtalène ; et
b) un porteur acceptable pharmaceutiquement pour la préparation d'une formulation pharmaceutique permettant de traiter des maladies oculaires associées à une étiologie immune.

2. Utilisation d'une quantité efficace thérapeutiquement d'un composé de la formule : 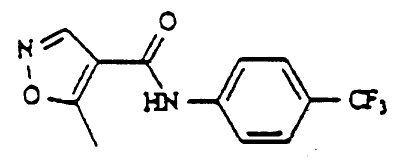 pour la préparation d'une formulation pharmaceutique permettant de traiter des maladies oculaires associées à une étiologie immune selon la revendication 1.

3. Utilisation de
a) une quantité efficace thérapeutiquement d'un composé ayant la formule : 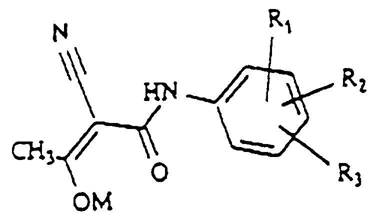 dans laquelle R₁, R₂ et R₃, qui peuvent être identiques ou différents, représentent chacun un halogène, tel que le chlore, le fluor, ou le brome, un groupe alkyle ayant 1, 2, 3 ou 4 atomes de carbone, un groupe alkoxy ayant 1, 2 ou 3 atomes de carbone, un groupe alkylthio de 1, 2 ou 3 atomes de carbone, ces groupes pouvant être substitués entièrement ou en partie par des atomes d'halogène identiques ou différents, tels que des atomes de fluor, de chlore, de brome ou d'iode ; un groupe nitro, cyano ou alkoxycarbonyle ayant 1, 2 ou 3 atomes de carbone dans la partie alkyle ; R₁ et R₂ représentent chacun en outre l'hydrogène, cas dans lequel, cependant, R₃ ne peut pas représenter le méthyle, mais peut représenter en outre un groupe phényle qui peut porter un ou deux atomes de fluor, de chlore, de brome ou d'iode, des groupes alkyle ayant 1, 2, 3 ou 4 atomes de carbone ou des groupes alkoxy ayant 1, 2 ou 3 atomes de carbone, ou peut représenter un groupe phénoxy qui peut porter un ou deux atomes de fluor, de chlore, de brome ou d'iode, des groupes alkyle ayant 1, 2 ou 3 atomes de carbone ou des groupes alkyoxy ayant 1, 2 ou 3 atomes de carbone, ou R₁ représente l'hydrogène, et R₂ et R₃ représentent ensemble un groupe méthylène-dioxy ou, conjointement avec l'anneau phényle auxquels ils sont liés, ils représentent un anneau de naphtalène, et dans laquelle M représente l'hydrogène, un métal alcalin, tel que le sodium ou le potassium, ou l'ammonium ; et
b) un porteur acceptable pharmaceutiquement pour la préparation d'une formulation pharmaceutique permettant de traiter des maladies oculaires associées à une étiologie immune selon la revendication 1.

4. Utilisation d'une quantité efficace thérapeutiquement d'un composé ayant la formule : 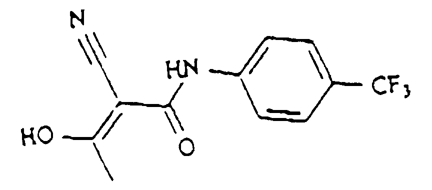 pour la préparation d'une formulation pharmaceutique permettant de traiter des maladies oculaires associées à une étiologie immune selon la revendication 3.

5. Utilisation d'une quantité efficace thérapeutiquement d'un composé ayant la formule : 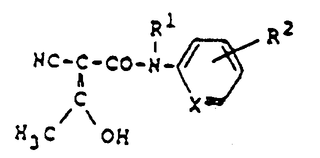 dans laquelle : 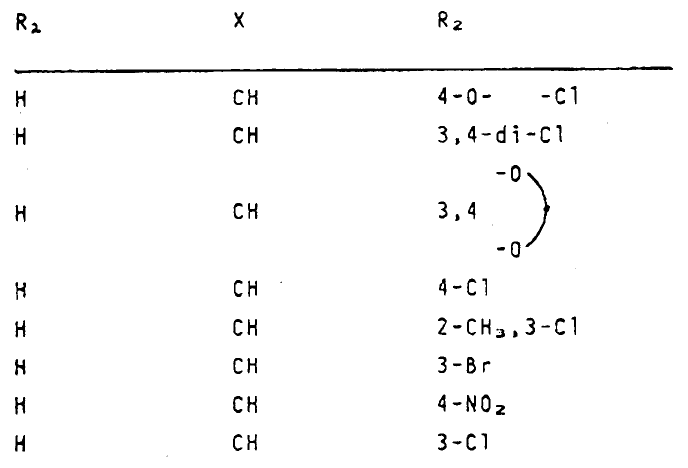 pour la préparation d'une formulation pharmaceutique permettant de traiter des maladies oculaires associées à une étiologie immune selon la revendication 3.

6. Utilisation selon une ou plusieurs des revendications 1 à 5, caractérisée en ce que le composé est fourni à une concentration comprise dans la plage allant de 0,0001 à 30 mg/kg/jour, de préférence à une concentration comprise dans la plage allant de 0,01 à 10,0 mg/kg/jour.

7. Utilisation selon une ou plusieurs des revendications 1 à 6, caractérisée en ce que la maladie oculaire associée à une étiologie immune est sélectionnée dans le groupe comprenant l'uvéite, la rétinite, l'allergie et l'oeil sec.

8. Utilisation selon une ou plusieurs des revendications 1 à 6, servant à prolonger la durée de vie d'une greffe de la cornée ou d'un autre tissu oculaire et servant d'ajouts chirurgicaux chez des patients qui sont atopiques ou souffrent d'une immunité déficiente.

9. Procédé pour la préparation d'une formulation pharmaceutique consistant en la composition suivante :
a) une quantité efficace thérapeutiquement d'un composé ayant la formule : 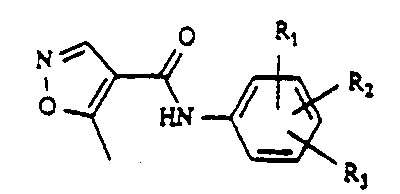 dans laquelle : R₁, R₂ et R₃, qui peuvent être identiques ou différents, représentent chacun un groupe alkyle ayant 1, 2 ou 3 atomes de carbone, un groupe alkoxy ayant 1, 2 ou 3 atomes de carbone, un groupe alkylthio de 1, 2 ou 3 atomes de carbone, ces groupes pouvant être substitués en partie ou entièrement par des atomes d'halogène identiques ou différents, tels que des atomes de fluor, de chlore, de brome ou d'iode ;
des atomes d'halogène, tels que le fluor, le chlore, le brome ou l'iode ;
un groupe nitro ;
un groupe cyano ;
des groupes alkoxycarbonyle ayant 1, 2 ou 3 atomes de carbone dans la partie alkyle, et dans laquelle R₁ et R₂ représentent chacun en outre l'hydrogène, cas dans lequel, cependant, R₃ ne peut pas représenter le méthyle, mais peut représenter en outre un groupe phényle qui peut porter un ou deux atomes de fluor, de chlore, de brome ou d'iode, des groupes alkyle ayant 1, 2 ou 3 atomes de carbone ou des groupes alkoxy ayant 1, 2 ou 3 atomes de carbone, ou peut représenter un groupe phénoxy qui peut porter un ou deux atomes de fluor, de chlore, de brome ou d'iode, des groupes alkyle ayant 1, 2 ou 3 atomes de carbone ou des groupes alkyoxy ayant 1, 2 ou 3 atomes de carbone, ou dans laquelle R₁ représente l'hydrogène, R₂ et R₃ représentent ensemble un groupe méthylène-dioxy ou, conjointement avec l'anneau phényle auxquels ils sont liés, ils représentent un anneau de naphtalène ; et
b) un porteur acceptable pharmaceutiquement,
pour obtenir la formulation pharmaceutique adaptée à une administration ophtalmique.

10. Procédé selon la revendication 9, caractérisé en ce que le composé est une quantité efficace thérapeutiquement d'un composé ayant la formule :

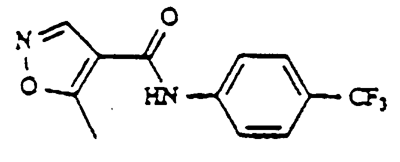

11. Procédé selon la revendication 9, caractérisé par le fait qu'il a la composition suivante :
a) une quantité efficace thérapeutiquement d'un métabolite d'un composé selon la revendication 9, ledit métabolite ayant la formule : 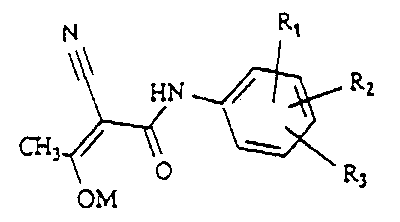 dans laquelle R₁, R₂ et R₃, qui peuvent être identiques ou différents, représentent chacun un halogène, tel que le chlore, le fluor, ou le brome, un groupe alkyle ayant 1, 2, 3 ou 4 atomes de carbone, un groupe alkoxy ayant 1, 2 ou 3 atomes de carbone, un groupe alkylthio de 1, 2 ou 3 atomes de carbone, ces groupes pouvant être substitués entièrement ou en partie par des atomes d'halogène identiques ou différents, tels que le fluor, le chlore, le brome ou l'iode ; un groupe nitro, cyano ou alkoxycarbonyle ayant 1, 2 ou 3 atomes de carbone dans la partie alkyle ; R₁ et R₂ représentent chacun en outre l'hydrogène, cas dans lequel, cependant, R₃ ne peut pas représenter le méthyle, mais peut représenter en outre un groupe phényle qui peut porter un ou deux atomes de fluor, de chlore, de brome ou d'iode, des groupes alkyle ayant 1, 2, 3 ou 4 atomes de carbone ou des groupes alkoxy ayant 1, 2 ou 3 atomes de carbone, ou peut représenter un groupe phénoxy qui peut porter un ou deux atomes de fluor, de chlore, de brome ou d'iode, des groupes alkyle ayant 1, 2 ou 3 atomes de carbone ou des groupes alkyoxy ayant 1, 2 ou 3 atomes de carbone, ou R₁ représente l'hydrogène, et R₂ et R₃ représentent ensemble un groupe méthylène-dioxy ou, conjointement avec l'anneau phényle auxquels ils sont liés, ils représentent un anneau de naphtalène, et dans laquelle M représente l'hydrogène, un métal alcalin, tel que le sodium ou le potassium, ou l'ammonium ; et
b) un porteur acceptable pharmaceutiquement,
pour obtenir la formulation pharmaceutique adaptée à une administration ophtalmique.

12. Procédé selon la revendication 11, caractérisé en ce que le métabolite présente la formule :

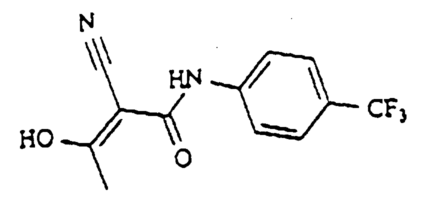

13. Procédé pharmaceutique selon la revendication 11, caractérisé en ce que le composé est une quantité efficace thérapeutiquement d'un composé ayant la formule : 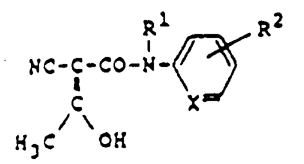 dans laquelle :

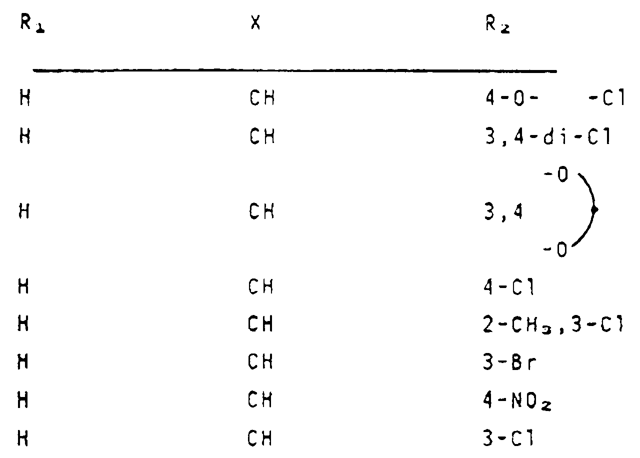

14. Utilisation d'
a) une quantité efficace thérapeutiquement d'un composé ayant la formule : 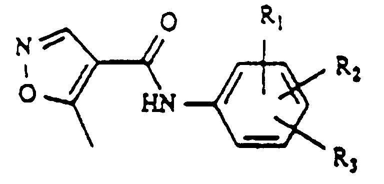 dans laquelle : R₁, R₂ et R₃, qui peuvent être identiques ou différents, représentent chacun un groupe alkyle ayant 1, 2 ou 3 atomes de carbone, un groupe alkoxy ayant 1, 2 ou 3 atomes de carbone, un groupe alkylthio de 1, 2 ou 3 atomes de carbone, ces groupes pouvant être substitués en partie ou entièrement par des atomes d'halogène identiques ou différents, tels que des atomes de fluor, de chlore, de brome ou d'iode ;
des atomes d'halogène tels que le fluor, le chlore, le brome ou l'iode ;
un groupe nitro ;
un groupe cyano ;
des groupes alkoxycarbonyle ayant 1, 2 ou 3 atomes de carbone dans la partie alkyle, et dans laquelle R₁ et R₂ représentent chacun en outre l'hydrogène, cas dans lequel, cependant, R₃ ne peut pas représenter le méthyle, mais peut représenter en outre un groupe phényle qui peut porter un ou deux atomes de fluor, de chlore, de brome ou d'iode, des groupes alkyle ayant 1, 2 ou 3 atomes de carbone ou des groupes alkoxy ayant 1, 2 ou 3 atomes de carbone, ou peut représenter un groupe phénoxy qui peut porter un ou deux atomes de fluor, de chlore, de brome ou d'iode, des groupes alkyle ayant 1, 2 ou 3 atomes de carbone ou des groupes alkyoxy ayant 1, 2 ou 3 atomes de carbone, ou dans laquelle R₁ représente l'hydrogène, R₂ et R₃ représentent ensemble un groupe méthylène-dioxy ou, conjointement avec l'anneau phényle auxquels ils sont liés, ils représentent un anneau de naphtalène ; et
b) un porteur acceptable pharmaceutiquement pour la préparation d'une formulation pharmaceutique, adaptée pour une administration ophtalmique, permettant de traiter des maladies oculaires associées à une étiologie immune.

15. Utilisation d'une quantité efficace thérapeutiquement d'un composé de la formule : 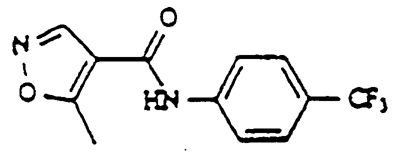 pour la préparation d'une formulation pharmaceutique, adaptée pour une administration ophtalmique, permettant de traiter des maladies oculaires associées à une étiologie immune selon la revendication 14.

16. Utilisation d'
a) une quantité efficace thérapeutiquement d'un composé ayant la formule : 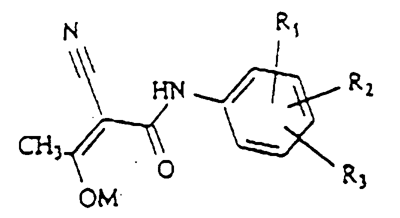 dans laquelle R₁, R₂ et R₃, qui peuvent être identiques ou différents, représentent chacun un halogène, tel que le chlore, le fluor, ou le brome, un groupe alkyle ayant 1, 2, 3 ou 4 atomes de carbone, un groupe alkoxy ayant 1, 2 ou 3 atomes de carbone, un groupe alkylthio de 1, 2 ou 3 atomes de carbone, ces groupes pouvant être substitués entièrement ou en partie par des atomes d'halogène identiques ou différents, tels que le fluor, le chlore, le brome ou l'iode ; un groupe nitro, cyano ou alkoxycarbonyle ayant 1, 2 ou 3 atomes de carbone dans la partie alkyle ; R₁ et R₂ représentent chacun en outre l'hydrogène, cas dans lequel, cependant, R₃ ne peut pas représenter le méthyle, mais peut représenter en outre un groupe phényle qui peut porter un ou deux atomes de fluor, de chlore, de brome ou d'iode, des groupes alkyle ayant 1, 2, 3 ou 4 atomes de carbone ou des groupes alkoxy ayant 1, 2 ou 3 atomes de carbone, ou peut représenter un groupe phénoxy qui peut porter un ou deux atomes de fluor, de chlore, de brome ou d'iode, des groupes alkyle ayant 1, 2 ou 3 atomes de carbone ou des groupes alkyoxy ayant 1, 2 ou 3 atomes de carbone, ou R₁ représente l'hydrogène, et R₂ et R₃ représentent ensemble un groupe méthylène-dioxy ou, conjointement avec l'anneau phényle auxquels ils sont liés, ils représentent un anneau de naphtalène, et dans laquelle M représente l'hydrogène, un métal alcalin, tel que le sodium ou le potassium, ou l'ammonium ; et
b) un porteur acceptable pharmaceutiquement pour la préparation d'une formulation pharmaceutique, adaptée pour une administration ophtalmique, permettant de traiter des maladies oculaires associées à une étiologie immune selon la revendication 14.

17. Utilisation d'une quantité efficace thérapeutiquement d'un composé ayant la formule : 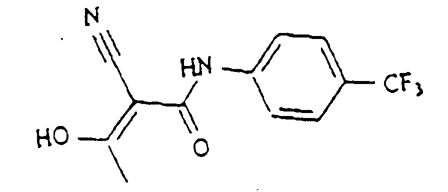 pour la préparation d'une formulation pharmaceutique, adaptée pour une administration ophtalmique, permettant de traiter des maladies oculaires associées à une étiologie immune selon la revendication 16.

18. Utilisation d'une quantité efficace thérapeutiquement d'un composé ayant la formule : 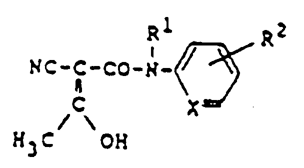 dans laquelle : 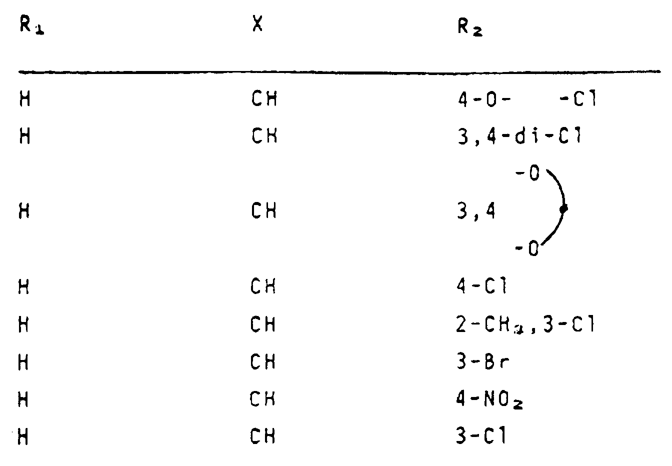 pour la préparation d'une formulation pharmaceutique, adaptée pour une administration ophtalmique, permettant de traiter des maladies oculaires associées à une étiologie immune selon la revendication 16.

19. Utilisation selon une ou plusieurs des revendications 14 à 18, caractérisée en ce que le composé est fourni à une concentration comprise dans la plage allant de 0,0001 à 30 mg/kg/jour, de préférence à une concentration comprise dans la plage allant de 0,01 à 10,0 mg/kg/jour.

20. Utilisation selon une ou plusieurs des revendications 14 à 18, caractérisée en ce que le composé est fourni localement dans l'oeil à une concentration comprise dans la plage allant de 0,05 à 10,0 % en poids.

21. Utilisation selon une ou plusieurs des revendications 14 à 18, caractérisée en ce que la maladie oculaire associée à une étiologie immune est sélectionnée dans le groupe comprenant l'uvéite, la rétinite, l'allergie et l'oeil sec.

22. Utilisation selon une ou plusieurs des revendications 14 à 18, servant en outre à prolonger la durée de vie d'une greffe de la cornée ou d'un autre tissu oculaire et servant d'ajouts chirurgicaux chez des patients qui sont atopiques ou souffrent d'une immunité déficiente.
